# EUROPÄISCHE PATENTSCHRIFT

(11) **EP 1 284 717 B1**
(45) Veröffentlichungstag und Bekanntmachung des Hinweises auf die Patenterteilung: **27.12.2006**
(21) Anmeldenummer: 01943457.0
(22) Anmeldetag: 29.05.2001
(51) Int. Cl.: A61K 9/16, A61K 9/48

(54) **FORMULIERUNG AUF HEPARIN-, GLYCOSAMINOGLYKAN- ODER HEPARINOIDBASIS UND VERWENDUNG DER FORMULIERUNG SOWIE DER FORMULIERUNGSGRUNDLAGE**
FORMULATION BASED ON HEPARIN, GLYCOSAMINOGLYCAN OR HEPARINOID, USE OF THE FORMULATION AND THE FORMULATION BASE
FORMULATION A BASE D'HEPARINE, DE GLYCOSAMINOGLYCANE OU D'HEPARINOIDE ET UTILISATION DE LADITE FORMULATION ET BASE DE LA FORMULATION

(30) Priorität: 30.05.2000 DE 10026699
(43) Veröffentlichungstag der Anmeldung: 26.02.2003
(73) Patentinhaber: Abbott GmbH & Co. KG, 65205 Wiesbaden (DE)
(72) Erfinder: ROSENBERG, Jörg, 67158 Ellerstadt (DE); BREITENBACH, Jörg, 68199 Mannheim (DE); HERR, Dieter, 67122 Altrip (DE); LAUX, Volker, 55128 Mainz (DE); HEGER, Robert, 69124 Heidelberg (DE)
(74) Vertreter: Kinzebach, Werner
(86) Internationale Anmeldenummer: PCT/EP2001/006115
(87) Internationale Veröffentlichungsnummer: WO 2001/091729

(56) Entgegenhaltungen:
- WO-A-01/01960
- WO-A-97/07786
- WO-A-99/44642
- US-A- 4 656 161
- US-A- 5 346 701
- US-A- 5 626 869
- US-A- 5 707 648
- YEH P-Y ET AL: "EFFECT OF MEDIUM-CHAIN GLYCERIDES ON PHYSIOGICAL PROPERTIES OF RABBIT INTESTINAL EPITHELIUM IN VITRO" PHARMACEUTICAL RESEARCH, NEW YORK, NY, US, Bd. 11, Nr. 8, 1994, Seiten 1148-1154, XP000886866 ISSN: 0724-8741

## Beschreibung

Die vorliegende Erfindung betrifft Formulierungen auf Basis wenigstens eines Heparins, Glycosaminoglykans oder Heparinoids und einer Formulierungsgrundlage mit einer Lipidkomponente und einer Polymerkomponente; die Verwendung dieser Formulierung als Arzneiform zur oralen Verabreichung wenigstens eines Heparins, Glycosaminoglykans oder Heparinoids; die Erfindung beschreibt auch ein Verfahren zur Herstellung der Formulierungen durch Vermischen der Formulierungskomponenten unter Bildung eines plastischen Gemisches und gegebenenfalls unter Herrichtung der Formulierungen als Arzneiform; und die Verwendung einer Formulierungsgrundlage bei der oralen Verabreichung von Heparinen, Glycosaminoglykanen oder Heparinoiden.

Die Vorzüge der für den Patienten an sich angenehmen oralen Verabreichung von Wirkstoffen werden oftmals gemindert durch Maßnahmen, die im Hinblick auf eine ausreichende Resorption eines Wirkstoffs im Gastrointestinaltrakt ergriffen werden müssen. So können verhältnismäßig hohe und auf mehrere Einzelverabfolgungen verteilte Tagesdosen erforderlich sein, um therapeutisch wirksame Blutspiegelwerte zu erhalten. Aus dem Bereich der Galenik sind Lösungsvorschläge bekannt, wonach die Wirkstoffe zusammen mit ausgewählten Hilfsstoffen formuliert werden. Es handelt sich dabei üblicherweise um nichtionogene Tenside mit recht hohen HLB-Werten, z.B. Cremophor®, Tween®, etc.

Obwohl diese Hilfsstoffe gemeinhin als chemisch indifferent bezeichnet werden, besitzen sie bekanntermaßen Nachteile, die sich insbesondere bei höheren Dosierungen durch lokale und/oder systemische Toxizität bemerkbar machen können.

Neben der lokalen Reizung, z.B. der Darmwand, können aufgrund der Resorption dieser Solubilisatoren unerwünschte Nebenwirkungen dieser Substanzen außerhalb des Gastrointestinaltraktes nicht ausgeschlossen werden.

Bekanntermaßen vermögen Emulsionen schwerlösliche Wirkstoffe kolloidal zu solubilisieren, wodurch die Bioverfügbarkeit solcher Wirkstoffe verbessert werden kann. So beschreibt US-A-5 707 648 eine Formulierung, die neben einer Ölphase, eine Polymerkomponente in Form von Polyethylenglykol, eine oberflächenaktive Substanz, Wasser und einen Wirkstoff, wie Heparin enthält. Parenteral verabreichbare Emulsionen verwenden in der Regel emulgierende Phospholipide, insbesondere Lecithine. Aufgrund der unzureichenden chemischen Beständigkeit der Phospholipide können diese Emulsionen allerdings erhebliche Lagerstabilitätsprobleme bereiten. Zudem ist die Herstellung solcher Emulsionen aufwendig. So kann es erforderlich sein, die Phospholipide in Wasser zusammen mit weiteren Emulsionsbestandteilen, beispielsweise Lipiden oder Lipidderivaten unter Hochdruck, d.h. bei mehreren 100 bar, zu homogenisieren.

Neben den zuvor beschriebenen flüssigen Emulsionen sind auch "feste" Emulsionen bekannt. Diese Formulierungen werden im Allgemeinen als selbstemulgierende Systeme bezeichnet, da sie sich in wässrigen Systemen unter Bildung einer Emulsion auflösen (vgl. M.O. Bachynsky et al., "Factors Affecting the Efficiency of a Self-Emulsifying Oral Delivery System", Drug Development and Industrial Pharmacy, 23 (8), (1997) 809-816). Auch hier kommen überwiegend die eingangs diskutierten solubilisationsfördernden Hilfsstoffe zur Anwendung, was die bekannten Nachteile mit sich bringt. Neben den vor allen zur Anwendung kommenden niedermolekularen Tensiden, z.B. Tween®, werden auch selbstemulgierende Systeme auf-Basis polymerer Glycerid-Tenside beschrieben (A.T.M. Serajuddin, "Bioavailability Enhancement of poorly Water-Soluble Drugs by Solid Dispersion in Surface Active and Self-Emulsifying Vehicles", Bulletin Technique Gattefossé, No.90, (1997.), S. 43-50). Diese polymeren Glyceride können aufgrund ihrer hohen HLB-Werte (z.B. Gelucire® 44/14 mit einem HLB-Wert von 14) als Tensid wirken. Wegen ihrer halbfesten Konsistenz müssen viele dieser Formulierungen in Gelatine-Kapseln abgefüllt werden. Dies gilt insbesondere für die Verwendung der in der Regel niedrig schmelzenden Glycerid-Tenside.

Aber auch leicht lösliche Wirkstoffe können unter Umständen schwer resorbierbar sein. Für solche Fälle schlägt die WO 99/42086 vor, Absorptionspromotoren mit HLB-Werten von mehr als 8 einzusetzen, vor allem polyglykosylierte Glyceride, z.B. das bereits genannte Gelucire®. Zu derart löslichen Wirkstoffen gehören mit den Heparinen Wirkstoffe, die vor allem auf dem Indikationsgebiet Thrombose seit vielen Jahren eine Standard-Therapie darstellen. Trotz intensivster Bemühungen, die bekanntermaßen extrem niedrige Bioverfügbarkeit von Heparinen nach oraler Gabe zu überwinden, ist es bisher nicht gelungen, ein marktreifes Präparat zu entwickeln. Zwar können nach oraler Verabfolgung von Heparin im APTT-Test dann Koagulationszeiten ermittelt werden, die denjenigen nach intravenöser Verabreichung vergleichbar sind, wenn Heparine in bestimmten Protinoid-Mikrosphären aus linearen thermischen Kondensationspolymeren gemischter Aminosäuren eingekapselt werden (vgl. US-A-4,925,673) oder gleichzeitig mit bestimmten synthetischen Lipid-Derivaten (z.B. β-(Hydroxybenzoyl)-aminooctansäure; J. Med. Chem. 41, (1998), 1163) gegeben werden. Nachteilig an diesen Formulierungen ist, dass extrem große Mengen an Hilfs- und Wirkstoffen appliziert werden müssen, so dass die Formulierungen in der Regel nur flüssig eingenommen werden können.

Die der vorliegenden Erfindung zugrunde liegende Aufgabe, Arzneiformen zur oralen Verabreichung von Heparinen, Glycosaminoglykanen oder Heparinoiden zur Verfügung zu stellen, wird überraschenderweise durch Formulierungen gelöst, deren Formulierungsgrundlage eine Lipidkomponente und eine Polymerkomponente aufweist.

Gegenstand der vorliegenden Erfindung sind daher Formulierungen auf Basis
i) wenigstens eines Heparins, Glycosaminoglykans oder Heparinoids und gegebenenfalls weiterer Wirkstoffe
   und einer Formulierungsgrundlage mit
ii) einer Lipidkomponente;
iii) einer Polymerkomponente; und
iv) gegebenenfalls weiteren pharmazeutisch akzeptablen Hilfsstoffen.

Der Begriff "Formulierung" meint im Rahmen der vorliegenden Erfindung ein aus den Komponenten i), ii), iii) und gegebenenfalls iv) zusammengesetztes Gemisch.

Der Begriff Heparin beschreibt eine Gruppe sulfatierter (sulfonierter) Mucopolysaccharide, die auch als Glycosaminoglykane bezeichnet werden. Strukturell sind Heparine gekennzeichnet durch Disaccharid-Einheiten aus α-1,4-glycosidisch verknüpften D-Glucosamin- und L-Iduronsäure-Einheiten sowie durch Disaccharid-Einheiten aus α-1,4-glycosidisch verknüpften D-Glucosamin- und D-Glucuronsäure-Einheiten. Sowohl die Stellung als auch die Anzahl der Sulfatgruppen (Sulfogruppen) ist variabel. Diese können sowohl über Sauerstoff (O-sulfatiert) als auch über Stickstoff (N-sulfatiert) gebunden sein. Häufig sind Iduronsäurereste 2-O-sulfatiert, Glucosaminreste N-sulfatiert und gegebenenfalls auch 6-O-sulfatiert. Glucuronsäurereste hingegen sind häufig nicht sulfatiert. Die Disaccharid-Einheiten sind wiederum α-1,4-glycosidisch miteinander zu Heparinmolekülen verbunden. Die Anzahl und Anordnung dieser Disaccharid-Einheiten kann ebenfalls variieren, so dass der Begriff Heparin eine Vielzahl strukturell unterschiedlicher Moleküle beschreibt, die beispielsweise elementaranalytisch oder anhand ihrer Kettenlänge, ihres Molekulargewichts oder ihrer Ladung unterschieden werden können. Vor allem werden mit dem Begriff Heparin Gemische strukturell unterschiedlicher Heparin-Moleküle der vorstehend beschriebenen Art (α-Heparine) bezeichnet, die gegebenenfalls auch noch weitere Bestandteile umfassen können, wie das sogenannte β-Heparin, auch Chondroitinsulfat B oder Dermatansulfat genannt, und/oder weitere Zellbestandteile, insbesondere Proteine. Solche Gemische lassen sich ebenfalls durch die zuvor genannten Parameter kennzeichnen, wobei es üblich ist, Mittelwerte und/oder Verteilungswerte beispielsweise Unter- und/oder Obergrenzen, anzugeben.

Heparine können als freie Säure, oder in Form physiologisch verträglicher Salze vorliegen. Bevorzugt sind die Natrium-, Calcium- und Magnesiumsalze.

Im Allgemeinen werden gegebenenfalls modifizierte Heparine natürlichen Ursprungs verabreicht. Heparine aus der Lunge, Leber oder Darmschleimhaut von Rindern oder Schweinen sind brauchbar, wobei Heparine aus Schweinedarmmucosa und aus Rinderlunge häufig verwendet werden.

Die Molekulargewichte von Heparinmolekülen liegen in der Regel im Bereich von 200 bis 30.000 Da. Erfindungsgemäß als Wirkstoff brauchbare Heparine können diesen gesamten Molekulargewichtsbereich oder aber nur Teile davon, insbesondere den niedermolekularen Bereich, abdecken. Bevorzugt sind sogenannte LMW-Heparine, d.h. Gemische aus Heparinmolekülen mit gewichtsmittleren Molekulargewichten von etwa 500 bis etwa 10.000 Da. Während unfraktionierte Heparine mit breiter Molekulargewichtsverteilung in der Regel gewichtsmittlere Molekulargewichte von etwa 10.000 bis 17.000 Da besitzen, liegen die gewichtsmittleren Molekulargewichte der LMW-Heparine deutlich darunter, in der Regel bei etwa 2000 bis 8000 und insbesondere bei etwa 3000 bis etwa 8000, etwa 4000 bis etwa 6000 oder etwa 4000 bis etwa 5000 Da.

Erfindungsgemäß brauchbare niedermolekulare Heparine werden durch Fraktionierung oder vorzugsweise Fragmentierung, d.h. Depolymerisation, von Heparinen mit breiterer Molekulargewichtsverteilung bzw. höherem gewichtsmittleren Molekulargewicht gewonnen. Als Ausgangsprodukte dienen insbesondere diejenigen Heparine, die aus natürlichen Quellen gewonnen werden und vor allem deren Calcium- oder Natriumsalze. Fraktioniert werden kann mittels Ethanolextraktion und fragmentiert vorzugsweise durch kontrollierte, partielle chemische oder enzymatische (z.B. Heparinase) oder physikalische (z.B. Ultraschall) Spaltung von Heparinen. Die chemische Spaltung gelingt beispielsweise mit Natriumnitrit und für die enzymatische Spaltung stehen spezifische Enzyme, in der Regel bakterielle Heparinasen, beispielsweise aus Flavobakterium, zur Verfügung.

Glycosaminoglykane sind negativ geladene Polysaccharide (Glykane), welche aus 1,4-verknüpften Einheiten von Disacchariden bestehen, in denen Uronsäure, z.B. D-Glucuronsäure und L-Iduronsäure, mit der 3- oder 4-Stellung eines N-acetylierten Aminozukkers (Glycosamins) glycosidisch verbunden ist.

Der Begriff Heparinoide beschreibt eine Gruppe von Substanzen mit heparinartiger Wirkung, d.h. Heparinoide hemmen die Blutgerinnung und die Entstehung von Thrombosen. Hierzu gehören beispielsweise sulfatierte pflanzliche Oligo- und Polysaccharide, z.B. aus Alginsäure, Pektinen, Xylanen, Stärken und Dextranen hergestellte Polysulfate oder sulfatierte tierische Glycosaminoglykane. Insbesondere zu nennen sind Pentosanpolysulfate, z.B. Natriumpentosan-, sulfonat, Xylansulfate, z.B: β-1,4-D-Xylan-2,3-bis(hydrogensulfat), Xylanpoly(hydrogensulfat) sowie Natriumsalze davon, Dextransulfate, Chitinsulfate, Chondroitinpolysulfate, auch Mucopolysaccharidpolyschwefelsäureester genannt, Polyvinylsulfonsäuren, auch Polyethylensulfonsäuren genannt, z.B. Natriumapolat, Polygalacturonsäuresulfat(methylestermethylglucosid), Alginatsulfate, z.B. Natriumalginatsulfat und Polymannuronsäuresulfat.

Heparinoide können entweder aus natürlichen Quellen gewonnen werden, oder sie werden semi- oder vollsynthetisch hergestellt, üblicherweise indem man die zuvor genannten pflanzlichen oder tierischen Polysaccharide sulfatiert, beispielsweise mit Chlorsulfonsäure umsetzt und freiwerdende Chlorwasserstoffsäure mit Basen neutralisiert.

Heparinen und Heparinoiden ist gemeinsam, dass sie zwar gut wasserlöslich sind, jedoch aus dem Gastrointestinaltrakt nur geringfügig resorbiert werden. Die unzureichende Resorbierbarkeit ist vor allem darauf zurückzuführen, dass Heparine und Heparinoide negativ geladen sind. Die erfindungsgemäße Formulierung eignet sich in besonders vorteilhafter Weise für eben diesen Wirkstofftyp, d.h. wasserlösliche und insbesondere negativ geladenen Substanzen, vor allem entsprechende sulfatierte Polysaccharide. Wasserlöslich sind Wirkstoffe im erfindungsgemäßen Sinne insbesondere dann, wenn sich 1 Teil des Wirkstoffs in höchstens 10 bis 30 Teilen, vorzugsweise in höchstens 1 bis 10 Teilen und insbesondere in weniger als 1 Teil Wasser lösen läßt.

Die Wirkstoffkomponente i) der erfindungsgemäßen, vorzugsweise festen Formulierungen enthält wenigstens ein Antikoagulans vom Heparin-, Glycosaminoglykan- oder Heparinoidtyp und kann weitere Antikoagulantien vom Heparin-, Glycosaminoglykan- oder Heparinoidtyp wie auch Antikoagulantien anderen Typs, wie Cumarinderivate, z.B. Warfarin, Phenprocoumon und Acenocoumarol, und weitere Wirkstoffe anderer Wirkung, wie Ergotamine und Dihydroergotamine, z.B. Dihydroergotaminmesilat, Thrombin-Inhibitoren, z.B. Argatroban und Melagatren, enthalten. Eine Ausführungsform der vorliegenden Erfindung sind Monopräparate, die als Wirkstoffkomponente ein Heparin, Glycosaminoglykan oder Heparinoid enthalten.

Die Wirkstoffkomponente macht in der Regel 1 bis 60 Gew.-%, vorzugsweise 5 bis 40 Gew.-% und insbesondere 10 bis 30 Gew.-% der Formulierung aus. Angaben in Gew.-% beziehen sich, sofern nicht anderes angegeben ist, auf das Gesamtgewicht der Formulierung.

Die Formulierungsgrundlage erfindungsgemäßer Formulierungen enthält pharmazeutisch akzeptable Hilfsstoffe, nämlich wenigstens ein Lipid, wenigstens ein Polymer und gegebenenfalls weitere pharmazeutisch akzeptable Hilfsstoffe. Pharmazeutisch akzeptabel sind die im Bereich der Pharmazie bekanntermaßen verwendbaren Hilfstoffe, insbesondere die in einschlägigen Arzneibüchern (z.B. DAB, Ph. Eur., BP, NF) gelisteten, und auch andere Hilfstoffe, deren Eigenschaften einer pharmazeutischen Anwendung nicht entgegenstehen.

Die Lipidkomponente erfindungsgemäßer Formulierungen enthält wenigstens ein Lipid, womit auch Lipidderivate und lipidhaltige Gemische bezeichnet sein sollen.

Der Begriff Lipid steht als Sammelbezeichnung für Fette und fettähnliche Substanzen. Die Fettähnlichkeit definiert sich insbesondere über das Löslichkeitsverhalten. Demnach sind fettähnliche Substanzen wie Fette selbst z.B. in Wasser praktisch unlöslich. Wasserunlöslich sind Substanzen im erfindungsgemäßen Sinne insbesondere dann, wenn zum Lösen von 1 Teil Substanz wenigstens 1000 bis 10000 Teile und vorzugsweise wenigstens 10000 Teile Wasser erforderlich sind. Man bezeichnet diese Substanzen auch als lipophil bzw. hydrophob.

Lipide, die sich der zu behandelnde Organismus zu eigen machen kann, also beispielsweise aufnehmen und gegebenenfalls metabolisieren kann, sind bevorzugt. In diesem Sinne realisieren diejenigen Lipide und Lipidderivate, die über den Gastrointestinaltrakt aufgenommen werden können, eine besondere Ausführungsform der vorliegenden Erfindung. Natürliche Lipide und Derivate natürlicher Lipide, die pflanzlichen oder tierischen Ursprungs sein können, sind bevorzugt.

Insbesondere bevorzugt ist wenigstens ein Lipid der Lipidkomponente ausgewählt unter körpereigenen Lipiden, vor allem Glyceriden und Fettsäuren bzw. Derivaten davon. Zu den körpereigenen Lipiden gehören vor allem Lipide auf Basis von Fetssäuren mit einer geradzahligen Anzahl an Kohlenstoffatomen.

Der Begriff Fettsäure bezeichnet eine Gruppe aliphatischer gesättigter oder ungesättigter Carbonsäuren. In der Regel sind es unverzweigte Ketten mit 6 bis 30, vorzugsweise 8 bis 22 und insbesondere 8 bis 18 Kohlenstoffatomen. Zu den gesättigten Fettsäuren gehören beispielsweise Capronsäure, Önanthsäure, Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure, Behensäure, Lignocerinsäure, Cerotinsäure und Melissinsäure. Die ungesättigten Fettsäuren können einfach oder mehrfach ungesättigt, insbesondere einfach, zweifach, dreifach, vierfach, fünffach oder sechsfach ungesättigt sein. Beispielsweise gehören zu den einfach ungesättigten Fettsäuren Palmitoleinsäure, Ölsäure und Erucasäure, zu den zweifach ungesättigten Fettsäuren Sorbinsäure und Linolsäure, zu den dreifach ungesättigten Fettsäuren Linolensäure und Elaeostearinsäure, zu den vierfach ungesättigten Fettsäuren Arachidonsäure, zu den fünffach ungesättigten Fettsäuren Clupanodonsäure und zu den sechsfach ungesättigten Fettsäuren Docosahexaensäure.

Bevorzugt sind einfach oder mehrfach ungesättigte Fettsäuren, vor allem Ölsäure, Palmitoleinsäure, Erucasäure, Linolsäure, Linolensäure.

Mit dem Begriff Glyceride bezeichnet man Ester des Glycerins. Je nach Anzahl der Estergruppen spricht man von Mono-, Di- und Triglyceriden. Der Säurerest eines Monoglycerids kann an 1- oder 2-Position sitzen und die Säurereste von Di- und Triglyceriden können gleich oder verschieden sein und in jeder erdenklichen Art auf die drei möglichen Positionen des Glycerins verteilt sein. Bei den Säureresten handelt es sich bevorzugt um die zuvor beschriebenen Fettsäuren. Beispielsweise gehören zu den Monoglyceriden Glycerinmonobehenat, Glycerinmonocaprat, Glycerinmonococoat, Glycerinmonoerucat, Glycerinmonoisostearat, Glycerinmonolanolat, Glycerinmonolaurat, Glycerinmonolinoleat, Glycerinmonomyristat, Glycerinmonooleat, Glycerinmonopalmitat, Glycerinmonoricinoleat, Glycerinmonostearat, zu den Diglyceriden Glycerindicaprylat, Glycerindilaurat, Glycerindimyristat, Glycerindioleat, Glycerindipalmitat und Glycerindistearat, zu den Triglyceriden Glycerintricaprylat, Glycerintrilaurat, Glycerintrimyristat, Glycerintrioctanoat, Glycerintrioleat, Glycerintriricinoleat und Glycerintristearat.

Bevorzugt sind Mono-, Di- und Triglyceride mit ungesättigten Fettsäureresten, insbesondere den erfindungsgemäß bevorzugt verwendbaren Fettsäureresten, vor allem Glycerinmonooleat, Glycerindioleat, Glycerintrioleat.

Vorzugsweise enthält die Lipidkomponente erfindungsgemäßer Formulierungen wenigstens eines der zuvor beschriebenen Lipide oder ein Gemisch aus wenigstens zwei der zuvor beschriebenen Lipide, wobei sie weitere dieser Lipide und auch andere Lipide enthalten kann.

Gemäß einer Ausführungsform der vorliegenden Erfindung besteht die Lipidkomponente aus einem der zuvor beschriebenen Lipide.

Gemäß einer anderen Ausführungsform der vorliegenden Erfindung besteht die Lipidkomponente aus einem Lipidgemisch aus wenigstens zwei der zuvor beschriebenen Lipide, Insbesondere aus einem Fettsäuregemisch, einem Glyceridgemisch oder einem Fettsäure/Glycerid-Gemisch.

Zu den Derivaten natürlicher Lipide, die pflanzlichen oder tierischen Ursprungs sein können, gehören vor allem diejenigen natürlichen Lipide, die chemisch und/oder physikalisch behandelt sind. Eine geeignete chemische Behandlung ist beispielsweise die Hydrierung ungesättigter Fettsäuren oder Fettsäurereste in Glyceriden. Eine geeignete physikalische Behandlung ist beispielsweise die Fraktionierung natürlicher Lipidgemische.

Zu den erfindungsgemäß verwendbaren Lipiden gehören auch lipidhaltige Naturstoffextrakte, die neben Lipid auch weitere Bestandteile enthalten können. Zu nennen sind hier vor allem die in einschlägigen Arzneibüchern gelisteten Lipide und Lipidgemische und auch Derivate davon, wie pflanzliche Öle oder tierische Fette, z.B. Olivenöl, Rizinusöl, Sesamöl, Erdnußöl, Mandelöl, Leinöl, Kakaobutter, Saffloröl, mittelkettige Triglyceride (Triglycerida mediocatenalia), Calcii behenas, Glyceroli monostearas, mittelkettige Partialglyceride (Partialglycerida mediocatenalia), höherkettige Partialglyceride (Partialglycerida longicatenalia), die gegebenenfalls auch hydriert oder raffiniert sein können, wie hydriertes Rizinusöl bzw. raffiniertes Rizinusöl. Auch hier sind Lipide mit einem Gehalt an ungesättigten Fettsäuren bzw. Fettsäureresten bevorzugt.

Gemäß einer besonderen Ausführungsform weist die Lipidkomponente einen HLB-Wert von höchstens 12, vorzugsweise von höchstens 8 und insbesondere von höchstens 5 auf. Das HLB-System (Hydrophil-Lipophil-Balance system) ordnet grenzflächenaktiven Stoffen numerische Zahlenwerte zu, lipophile Substanzen erhalten niedrige, hydrophile höhere HLB-Werte (Fiedler, H.B., Lexikon der Hilfsstoffe für Pharmazie, Kosmetik, und angrenzende Gebiete, 4. Aufl., Aulendorf: ECV-Editio-Cantor-Verlag (1996)). Insbesondere ist die Lipidkomponenete nicht oder nur schlecht in Wasser löslich. Demnach läßt sich diese Ausführungsform vor allem mit den zuvor genannten Fettsäuren und Glyceriden verwirklichen.

Gemäß einer weiteren bevorzugten Ausführungsform weist die Lipidkomponente einen Schmelzpunkt von höchstens 50°C, vorzugsweise von höchstens 40°C und insbesondere von weniger als 30°C auf. Demnach läßt sich diese Ausführungsform vor allem mit Fettsäuren.wie.Tridecansäure, Laurinsäure, Elaeostearinsäure, vorzugsweise Undecansäure, Caprinsäure, Erucasäure, insbesondere Pelargonsäure, Caprylsäure, Önanthsäure, Capronsäure, Isostearinsäure, Ölsäure, palmitoleinsäure, Linolsäure, Linolensäure, Arachidonsäure, Clupanodonsäure und Docosahexaensäure, und Glyceriden, wie Glycerinmonolaurat, Glycerinmonolinoleat, Glycerinmonooleat, Glycerinmonopalmitat, Glycerinmonoricinoleat, Glycerindioleat, Glycerintrioleat und Glycerintriricinoleat, verwirklichen.

Insbesondere bevorzugt ist es, dass wenigstens ein Teil der Lipidkomponente und wenigstens ein Teil der Polymerkomponente in den erfindungsgemäßen Formulierungen eine molekulardisperse Verteilung ausbilden. Ist der Lipidanteil größer als der Polymeranteil, so spricht man von einer molekulardispersen Verteilung des Polymers im Lipid. Vorzugsweise ist der Lipidanteil geringer als der Polymeranteil; dann spricht man von einer molekulardispersen Verteilung des Lipids im Polymer.

Der Begriff "molekulardispers" ist dem Fachmann bekannt und beschreibt im Wesentlichen Systeme, in denen eine Substanz, im vorliegenden Fall wenigstens ein Teil und vorzugsweise der überwiegende Teil der Lipid- bzw. Polymerkomponente, in einem Lösungsmittel in homogener Verteilung dispergiert ist. Das Lösungsmittel bildet dabei in der Regel eine Matrix, die erfindungsgemäß von der Polymer- bzw. Lipidkomponente oder zumindest von einem überwiegenden Teil von der Polymer- bzw. Lipidkomponente gebildet wird. Der Anteil an Lipidkristallen in einer erfindungsgemäßen Formulierung liegt in der Regel unter 12% und insbesondere unter 5%. Angaben zu Kristallanteilen beziehen sich auf die Gesamtmenge der jeweiligen Komponente.

Gemäß einer besonderen Ausführungsform sind molekulardisperse Systeme fest, sie werden dann als feste Lösungen bezeichnet.

Eine erfindungsgemäße Formulierung, die im Wesentlichen frei von Lipidkristallen ist, stellt eine besondere Ausführungsform der vorliegenden Erfindung dar. Dieser Zustand entspricht der maximal möglichen Homogenisierung des Lipids bzw. Polymers in der Matrix. In der molekulardispersen Verteilung ist das System grenzflächenfrei.

Gemäß einer weiteren besonderen Ausführungsform liegt wenigstens ein Teil der Wirkstoffkomponente als molekulardisperse Verteilung vor. Der Anteil an Wirkstoffkristallen in einer erfindungsgemäßen Formulierung liegt in der Regel unter 12% und insbesondere unter 5%. Zu diesen Formulierungen gehören insbesondere diejenigen, die im Wesentlichen frei von Wirkstoffkristallen sind. Dieser Zustand entspricht der maximal möglichen Homogenisierung des Wirkstoffs in der Formulierungsgrundlage.

Erfindungsgemäße Formulierungen, die im Wesentlichen frei von Lipid- und Wirkstoffkristallen sind, und vor allem solche, in denen im Wesentlichen kein Bestandteil kristalline Anteile aufweist (im wesentlichen amorphe bzw. kristallfreie Formulierungen), stellen eine weitere besondere Ausführungsform der vorliegenden Erfindung dar. Dieser Zustand entspricht der maximal möglichen Homogenisierung der Formulierungskomponenten. In der molekulardispersen Verteilung ist die Formulierung grenzflächenfrei.

Der Zustand solcher molekulardispersen Verteilungen, insbesondere der fester Lösungen, läßt sich mit bekannten analytischen Verfahren untersuchen, z.B. mit der Differential Scanning Calorimetry (DSC) oder mit Weitwinkelröntgenstreuung-Messungen (WAXS-Messungen). Liegt eine moleulardisperse Verteilung vor, so fehlt der bei der DSC-analytischen Messung der kristallinen Reinsubstanz auftretende, in der Regel endotherme Schmelzpeak. Eine weitere Möglichkeit zur Kennzeichnung einer molekulardispersen Verteilung ist die Intensitätsverminderung und/oder Abwesenheit typischer Röntgenbeugungs-Signale bei WAXS-Analytik.

Der Anteil der Lipidkomponente an der Formulierung beträgt in der Regel 3 bis 50 Gew.-%, vorzugsweise 6 bis 35 Gew.-% und insbesondere 11 bis 30 Gew.-%.

Ein Kriterium für die Ermittlung der optimalen Menge Lipid ist die Homogenität der erfindungsgemäßen Formulierung in der Schmelze. Insbesondere mit Blick auf die Obergrenze sollte eine homogene Einarbeitung des Lipids in die Schmelze ohne Phasenseparation gewährleistet sein.

Gemäß einer besonderen Ausführungsform der vorliegenden Erfindung beträgt der Anteil der Lipidkomponente bezogen auf die Polymerkomponente höchstens 40 Gew.-%, vorzugsweise höchstens 30 Gew.-% und insbesondere höchstens 25 Gew.-%.

Die Polymerkomponente der erfindungsgemäßen Formulierungen kann auch als polymeres Bindemittel begriffen werden, das zumindest teilweise eine Polymermatrix bildet. Bindemittel im Sinne der Erfindung sind feste schmelzbare Lösungsmittel. Die Polymermatrix dient vor allem zur Aufnahme und insbesondere zum Lösen wenigstens eines Teils der Lipidkomponente. Vorzugsweise kommt es dabei zur Ausbildung von molekulardispersen Verteilungen. Diesbezüglich wird auf obige Ausführungen in Zusammenhang mit der Lipidkomponente Bezug genommen.

Vorzugsweise ist die Polymerkomponente in physiologischer Umgebung, d.h. insbesondere im Gastrointestinaltrakt, vor allem im oberen Dünndarmbereich und bevorzugt im Duodenum, zumindest teilweise löslich oder quellbar. Unter Quellung versteht man im Wesentlichen einen Vorgang, bei dem sich Volumen und/oder Gestalt eines Festkörpers, beispielsweise einer erfindungsgemäßen festen Formulierung, bei Einwirkung von Flüssigkeiten, Dämpfen und Gasen, erfindungsgemäß also in der Regel bei Einwirkung von Körperflüssigkeiten und insbesondere denen des Gastrointestinaltraktes, ändern. Quellbar bzw. löslich sind vor allem hydrophile Polymere, die Wasser zumindest oberflächlich anzulagern und/oder zwischen die Polymerketten, vornehmlich durch Absorption, aufzunehmen vermögen. Eine begrenzte Quellung führt in der Regel zur Gelbildung, weshalb erfindungsgemäß brauchbare, begrenzt quellbare Polymere unter den gemeinhin als Gelbildnern bekannten Polymeren ausgewählt werden können. Eine unbegrenzte Quellung führt in der Regel zur Ausbildung von Lösungen oder kolloidalen Lösungen, weshalb erfindungsgemäß brauchbare unbegrenzt quellbare Polymere unter den in physiologischer Umgebung, insbesondere in den Körperflüssigkeiten des Gastrointestinaltraktes, zumindest kolloidal löslichen Polymeren ausgewählt werden können. Insbesondere mit Blick auf den Gastrointestinaltrakt ist zu berücksichtigen, dass die physiologischen Bedingungen, vor allem der pH-Wert, örtlich unterschiedlich sein können. Ist es beispielsweise bevorzugt, dass der Wirkstoff vornehmlich im Duodenum aufgenommen wird, so kann es von Vorteil sein, daß die Polymerkomponente unter den im Duodenum herrschenden Bedingungen quellbar ist. Insbesondere kann es von Vorteil sein, wenn in vorgeschalteten Abschnitten des Gastrointestinaltraktes, insbesondere im Magen nur eine geringe oder vorzugsweise im Wesentlichen keine Quellung erfolgt. Es sei allerdings schon an dieser Stelle bemerkt, dass ein solches Verhalten erfindungsgemäßer Formulierungen nach Verabfolgung auch mit anderen Mitteln gewährleistet werden kann, im vorstehend geschilderten Fall beispielsweise mit magensaftresistenten Überzügen oder mehrschichtigen Formulierungen, in denen meist innenliegende wirkstoffhaltige Schichten erst am erwünschten Ort einer Quellung oder Lösung ausgesetzt sind.

Gemäß einer besonderen Ausführungsform bildet die Polymerkomponente unter den Anwendungsbedingungen der Formulierung keine Micellen aus. Eine CMC (kritische Micellbildungskonzentration) wird nicht erreicht.

Verfahrenstechnisch sind Polymerkomponenten bevorzugt, die thermoplastisch verarbeitbar sind.

Vorzugsweise ist wenigstens ein Polymer der Polymerkomponente ausgewählt unter:
Synthetischen Polymeren, wie Polyvinyllactamen, insbesondere, Polyvinylpyrrolidon (PVP); Copolymeren von Vinyllactamen, wie N-Vinylpyrrolidon, N-Vinylpiperidon und N-Vinyl-ε-caprolactam, aber insbesondere N-Vinylpyrrolidon, mit (Meth)acrylsäure und/oder (Meth)acrylsäureestern, wie langkettigen (Meth)acrylaten, z.B. Stearyl(meth)acrylat, Dialkylaminoalkyl(meth)acrylaten, die gegebenenfalls quaternisiert sein können, und Maleinsäureanhydrid, Vinylestern, insbesondere Vinylacetat, Vinylformamid, Vinylsulfonsäure oder quaternisiertem Vinylimidazol; Copolymerisaten von Vinylacetat und Crotonsäure; teilverseiftem Polyvinylacetat; Polyvinylalkohol; (Meth)acrylharzen, wie Polyhydroxyalkyl(meth)acrylaten, Poly(meth)acrylaten, Acrylatcopolymeren, z.B. aus Acrylsäurealkylestern mit (Meth)acrylsäure, und Copolymerisaten von Dimethylaminoethylacrylaten und Methacrylestern (z.B. Eudragit-Typen); Polyalkylenglykolen, wie Polypropylenglykolen und Polyethylenglykolen, vorzugsweise mit Molekulargewichten oberhalb von 1000, besonders bevorzugt oberhalb von 2000 und ganz besonders bevorzugt oberhalb von 4000 (z.B. Polyethylenglykol 6000); Polyalkylenoxiden, wie Polypropylenoxiden und vor allem Polyethylenoxiden, vorzugsweise hochmolekular, vor allem mit gewichtsmittleren Molekulargewichten von mehr als 100000; Copolymerisaten von Methylmethacrylat und Acrylsäure; Polyacrylamiden, Polyvinylformamid (gegebenenfalls partiell oder vollständig hydrolysiert);
modifizierten natürlichen Polymeren, z.B. modifizierten Stärken und modifizierten Cellulosen, wie Celluloseestern und bevorzugt Celluloseethern, z.B. Methylcellulose und Ethylcellulose, Hydroxyalkylcellulosen, insbesondere Hydroxypropylcellulose, Hydroxyalkyl-Alkylcellulosen, insbesondere HydroxypropylMethylcellulose oder Hydroxypropyl-Ethylcellulose, Cellulosephthalaten, insbesondere Celluloseacetatphthalat und Hydroxypropylmethylcellulosephthalat; und
natürlichen oder überwiegend natürlichen Polymeren, wie Gelatine, Polyhydroxyalkanoaten, z.B. Polyhydroxybuttersäure und Polymilchsäure, Polyaminosäuren, z.B. Polylysin, Polyasparagin, Polydioxane und Polypeptiden, und Mannanen, insbesondere Galactomannanen.

Hiervon sind die modifizierten natürlichen und vor allem die synthetischen Polymere bevorzugt.

Insbesondere bevorzugt ist wenigstens ein Polymer der Polymerkomponente ausgewählt unter Polyvinylpyrrolidonen, Vinylpyrrolidon-Vinylacetat-Copolymeren, Hydroxyalkylcellulosen, Hydroxyalkyl-Alkylcellulosen, Cellulosephthalaten, Polyalkylenglycolen, (Meth)acrylharzen: beispielsweise die unter der Handelsbezeichnung Kollidon® geführten Polyvinylpyrrolidone mit gewichtsmittleren Molekulargewichten von etwa 2000 bis etwa 1,5 x 10⁶, beispielsweise das unter der Handelsbezeichnung Kollidon® 17 PF geführte Polyvinylpyrrolidon mit einem gewichtsmittleren Molekulargewicht von etwa 7000 bis etwa 11000; Vinylpyrrolidon-Vinylacetat-Copolymere insbesondere mit einem Verhältnis Vinylpyrrolidon:Vinylacetat von etwa 30 zu etwa 70 bis etwa 70 zu etwa 30, beispielsweise das unter der Handelsbezeichnung Kollidon® VA 64 geführte Produkt mit einem Verhältnis Vinylpyrrolidon:Vinylacetat von etwa 60 zu etwa 40; Hydroxyalkylcellulosen mit 1 bis 3 Kohlenstoffatomen im Alkylteil, insbesondere Hydroxypropylcellulose, beispielsweise die unter der Handelsbezeichnung Klucel® geführte Hydroxypropylcellulose; Hydroxyalkyl-Alkylcellulosen mit 1 bis 3 Kohlenstoffatomen in den Alkylteilen, insbesondere Hydroxypropylmethylcellulose, beispielsweise die unter der Handelsbezeichnung Methocel® geführten Ethyl-, Hydroxyethyl-, Hydroxypropyl und Carboxymethylethergruppen enthaltenden Methylcellulose- und Methylcellulosederivatgemische, Cellulosephthalate, insbesondere Hydroxypropylmethylcellulosephthalat, Polyalkylenglycole mit 2 und/oder 3 Kohlenstoffatomen im Alkylenteil, insbesondere Polyethylenglycole, beispielsweise die unter der Handelsbezeichnung Lutrol® geführten Polyethylenglycole mit gewichtsmittleren Molekulargewichten von etwa 2000 bis etwa 20000, und Polypropylenglycole, Copolymerisate auf Basis von Dimethylaminoethylmethacrylat und Methacrylsäureestern wie Methacrylsäuremethylester und Methacrylsäurebutylester, beispielsweise die unter der Handelsbezeichnung Eudragit® E geführten Acrylharze auf Basis von Dimethylaminoethylmethacrylat, Methyl- und Butyl(meth)acrylat mit gewichtsmittleren Molekulargewichten von etwa 150000, Copolymerisate mit anionischem Charakter auf Basis von Methacrylsäure und Methacrylsäuremethylester, beispielsweise die unter den Handelsbezeichnungen Eudragit® L bzw. S geführten Acrylharze mit gewichtsmittleren Molekulargewichten von 250000 bzw 135000.

Ganz besonders bevorzugt sind die vorstehend genannten Polyvinylpyrrolidone und Cellulosederivate, vor allem Kollidon® VA 64, niedermolekulare Hydroxypropylcellulose, z.B. Klucel®EF mit gewichtsmittleren Molekulargewichten von etwa 45000 bis etwa 70000 bzw. etwa 80000, und Hydroxypropylmethylcellulose, z.B. Methocel® E3, E5 und E7.

Die Polymerkomponente erfindungsgemäßer Formulierungen enthält vorzugsweise wenigstens eines der zuvor beschriebenen Polymere. Sie kann weitere dieser Polymere und/oder andere Polymere enthalten. Über die Art des gewählten Polymers oder der Abmischung unterschiedlicher Polymere können die Eigenschaften der erfindungsgemäßen Formulierung variiert werden. Insbesondere kann auf diese Weise die Wirkstoff-Freisetzung gesteuert werden.

Gemäß einer Ausführungsform der vorliegenden Erfindung besteht die Polymerkomponente aus einem der zuvor beschriebenen Polymere. Gemäß einer anderen Ausführungsform der vorliegenden Erfindung besteht die Polymerkomponente aus einem Gemisch aus wenigstens zwei der zuvor beschriebenen Polymere.

Vorteilhaft für die Verwendung als polymeres Bindemittel sind solche Polymere, die einen K-Wert (nach H. Fikentscher, Cellulose-Chemie 13 (1932), S. 58-64 und 71-74) im Bereich zwischen 10 und 100, insbesondere zwischen 15 und 80 aufweisen.

Der Anteil der Polymerkomponente an der erfindungsgemäßen festen Formulierung beträgt in der Regel 5 bis 96 Gew.-%, vorzugsweise 10 bis 80 Gew.-% und insbesondere 20 bis 70 Gew.-%.

Erfindungsgemäße Formulierungen können neben Polymer- und Lipidkomponente weitere pharmazeutisch akzeptable Hilfsstoffe enthalten (Hilfsstoffkomponente iv). Solche Hilfsstoffe können die Herstellung der Formulierung erleichtern und/oder deren Eigenschaften modulieren. Art und Menge werden vorteilhafterweise so gewählt, daß sie die Ausbildung der speziellen Eigenschaften der erfindungsgemäßen Formulierungen und einer gegebenenfalls vorhandenen molekulardispersen Verteilung, insbesondere einer festen Lösung nicht beinträchtigen bzw. nicht zu einer Destabilisierung dieses Systems beitragen.

Hilfsstoffe sind z.B. übliche galenische Hilfsstoffe, deren Gesamtmenge bis zu 100 Gew.-%, bezogen auf die Polymerkomponente, betragen kann, z.B.

Füllstoffe, wie Zuckeralkohole, z.B. Mannit, Sorbit, Xylit und Isomalt (vgl. DE 195 36 394), Talkum, Saccharose, Lactose, Getreide- oder Maisstärke, Kartoffelmehl, soweit vorhanden insbesondere in einer Konzentration von 0,02 bis 50, vorzugsweise 0,20 bis 20 Gew.-%, bezogen auf das Gesamtgewicht des Gemisches;

Schmiermittel, Gleitmittel und Trennmittel wie Magnesium-, Aluminium- und Calciumstearat, Talkum und Silicone, sowie tierische oder pflanzliche Fette, insbesondere in hydrierter Form und solche, die bei Raumtemperatur fest sind. Diese Fette haben vorzugsweise einen Schmelzpunkt von 30°C oder höher. Verfahrenstechnisch im Hinblick auf die Schmelzextrusion bevorzugt sind - wie in der DE 197 31 277 bechrieben - Triglyceride der C₁₂-, C₁₄-, C₁₆- und C₁₈-Fettsäuren, oder - zwecks Verbesserung der Verarbeitungseigenschaften - Lecithin, wie im Zusammenhang mit der Extrusion einer Isomalt enthaltenden Polymer-Wirkstoffschmelze in der DE 195 36 394 beschrieben ist. Auch Wachse, wie Carnaubawachs, sind brauchbar. Diese Fette und Wachse können vorteilhaft alleine oder zusammen mit Mono- und/oder Diglyceriden oder Phosphatiden, insbesondere Lecithin, zugemischt werden. Die Mono- und Diglyceride stammen vorzugsweise von den oben erwähnten Fettsäuretypen ab. In der Regel erfüllen die erfindungsgemäß vorhandenen Lipide die Funktion dieser Hilfsstoffe, so daß nur geringe Mengen und vorteilhafterweise keine Schmiermittel, Gleitmittel und Trennmittel als Hilfsstoffe der Formulierung zugesetzt werden. Soweit vorhanden, beträgt die Gesamthilfsstoffmenge an Schmier- und Trennmitteln vorzugsweise 0,1 bis 10 Gew.-% und insbesondere 0,1 bis 1 Gew.% bezogen auf das Gesamtgewicht des Gemisches;

Fließmittel, z.B. Kieselerden, insbesondere die unter der Handelsbezeichnung Aerosil® geführten hochreinen Siliziumdioxide, soweit vorhanden insbesondere in einer Menge von 0,1 bis 5 Gew.-%, bezogen auf das Gesamtgewicht des Gemisches;

Farbstoffe, wie Azofarbstoffe, organische oder anorganische Pigmente oder Farbstoffe natürlicher Herkunft, wobei anorganische Pigmente soweit vorhanden in einer Konzentration von 0,001 bis 10, vorzugsweise 0,5 bis 3 Gew.-%, bezogen auf das Gesamtgewicht des Gemisches bevorzugt sind;

Stabilisatoren, wie Antioxidanzien, Lichtstabilisatoren, Hydroperoxid-Vernichter, Radikalfänger, Stabilisatoren gegen mikrobiellen Befall;

Weichmacher, insbesondere die unten beschriebenen.

Ferner können Netz-, Konservierungs-, Spreng-, Adsorptions- und Formentrennmittel sowie Tenside, insbesondere anionische und nicht-ionische, wie z.B. Seifen und seifenähnliche Tenside, Alkylsulfate und -sulfonate, Salze von Gallensäuren, alkoxylierte Fettalkohole, alkoxylierte Alkylphenole, alkoxylierte Fettsäuren und Fettsäureglycerinester, die alkoxyliert sein können, und Solubilisierungsmittel, wie Cremophor (polyethoxyliertes Rizinusöl), Gelucire, Vitamin E-TPGS und Tween (ethoxylierte Sorbitanfettsäureester), zugesetzt werden (vgl. z.B. H. Sucker et al. Pharmazeutische Technologie, Thieme-Verlag, Stuttgart 1978). Da die erfindungsgemäßen Formulierungen beim Kontakt mit Wasser oder wäßrigen Lösungsmitteln Emulsionen ausbilden, kann ein Zusatz eines oberflächenaktiven Hilfsstoffs, insbesondere von Stoffen mit hohen HLB-Werten, vor allem von mehr als 8, 10 und insbesondere von über 15, gering, in der Regel in Mengen von weniger als 1 Gew.-%, gehalten werden. Vorteilhafterweise kann auf einen solchen Zusatz verzichtet werden.

Unter Hilfsstoffen im Sinne der Erfindung sind auch Substanzen zur Herstellung einer festen Lösung mit dem pharmazeutischen Wirkstoff zu verstehen. Diese Hilfsstoffe sind beispielsweise Pentaerythrit und Pentaerythrittetraacetat, Harnstoff, Phosphatide, wie Lecithin, sowie Zuckeralkohole, wie Xylit und Mannit, Zitronen- und Bernsteinsäure, Gallensäuren, Stearine und andere, wie z.B. bei J. L. Ford, Pharm. Acta Helv. 61, (1986), S. 69-88, angegeben.

Als pharmazeutische Hilfsstoffe gelten auch Zusätze von Basen und Säuren zur Steuerung der Löslichkeit eines Wirkstoffes (siehe beispielsweise K. Thoma et al., Pharm. Ind. 51, (1989), S. 98-101).

Hilfsstoffe im erfindungsgemäßen Sinn sind auch arzneiformspezifische, d.h. auf eine bestimmte Arzneiform, insbesondere perorale und vor allem Tabletten und Kapseln ausgerichtete Vehikel, auch niedrigschmelzende bzw. flüssige Hilfsstoffe, wie Polyalkylenglykole mit niedrigem Molekulargewicht, insbesondere Polyethylenglykol und/oder Polypropylenglykol mit gewichtsmittleren Molekulargewichten von weniger als 1.000, Wasser oder geeignete wässrige Systeme.

Zugesetzt werden können auch Hilfsstoffe, wie Geschmackskorrigentien und Geruchsmaskierungsmittel, insbesondere Süßstoffe und Aromen.

Eine diesbezügliche Ausgestaltung beruht auf fachrmännischem Wissen, wie es beispielsweise in Fiedler, H.B., Lexikon der Hilfsstoffe für Pharmazie, Kosmetik, und angrenzende Gebiete, 4.Aufl., Aulendorf: ECV-Editio-Cantor-Verlag (1996), dargestellt ist.

Die Hilfsstoffkomponente erfindungsgemäßer fester Formulierungen enthält vorzugsweise wenigstens einen der zuvor beschriebenen weiteren Hilfsstoffe. Sie kann weitere dieser Hilfsstoffe und/oder andere Hilfsstoffe enthalten.

Eine Ausführungsform der vorliegenden Erfindung sind Formulierungsgrundlagen mit Hilfsstoffkomponente. In diesem Fall kann der Anteil an weiteren pharmazeutisch akzeptablen Hilfsstoffen in erfindungsgemäßen Formulierungen bis zu 91 Gew.-%, vorzugsweise bis zu 60 Gew.-% und insbesondere bis zu 40 Gew.-% betragen.

Eine besondere Ausführungsform der vorliegenden Erfindung sind Formulierungen, die
i) niedermolekulares Heparin, insbesondere solches mit einem gewichtsmittleren Molekulargewicht von etwa 500 bis etwa 10000 ;
ii) wenigstens eine ungesättigte Fettsäure, die vorzugsweise ausgewählt ist unter Ölsäure, Linolsäure und/oder Linolensäure, oder entsprechenden Mono- oder Diglyceriden;
iii) wenigstens ein unter Polyvinylpyrrolidonen, Vinylpyrrolidon-Copolymerisaten insbesondere mit Vinylacetat, oder Cellulosederivaten, insbesondere Hydroxypropylcellulosen und Hydroxypropylmethylcellulosen ausgewähltes Polymer; und
iv) gegebenenfalls weitere Hilfsstoffe, beispielsweise ein Fließmittel,
enthalten.

Die erfindungsgemäßen Formulierungen enthalten vorzugsweise weniger als 5 Gew.-% und insbesondere weniger als 1 Gew.-% Wasser. Eine besondere Ausführungsform stellen im wesentlichen wasserfreie Formulierungen dar.

Die erfindungsgemäßen Formulierungen sind vorzugsweise von fester Konsistenz. Der Begriff "fest" besitzt hier die in einschlägigen Arzneibüchern in Zusammenhang mit Arzneizubereitungen zugeordnete Bedeutung. Des weiteren können erfindungsgemäße Formulierungen auch von halbfester oder flüssig-viskoser Konsistenz sein. Auch die Begriffe "halbfest" und "flüssig-viskos" besitzen im Rahmen der vorliegenden Erfindung die in einschlägigen Arzneibüchern, in Zusammenhang mit Arzneizubereitungen zugeordneten Bedeutungen. Beispielsweise können bei verhältnismäßig hohen Anteilen an Lipiden und vor allem niedrigschmelzenden Lipiden, erfindungsgemäße Formulierungen von halbfester Konsistenz sein. Eine halbfeste und gewünschtenfalls auch flüssig-viskose Konsistenz kann bekanntermaßen auch durch den Zusatz geeigneter Hilfstoffe, insbesondere niedrigschmelzender oder flüssiger vehikel, erzielt werden.

Gegenstand der vorliegenden Erfindung ist daher auch die Verwendung erfindungsgemäßer Formulierungen als Arzneiform bei der oralen Verabreichung wenigstens eines Heparins, Glycosaminoglykans oder Heparinoids.

Demnach finden erfindungsgemäße Formulierungen vornehmlich Anwendung im pharmazeutischen, sowohl im human- als auch tiermedizinischen Bereich. In diesem Sinne finden die Formulierungen Anwendung als oder in Arzneiformen, d.h. die erfindungsgemäßen Formulierungen weisen, erforderlichenfalls zusammen mit weiteren Hilfsstoffen, zweckmäßige, der pharmazeutischen Praxis entsprechende Formen auf.

So bezeichnet der Begriff "Arzneiform" eine beliebige Darreichungsform zur Verabfolgung von Wirkstoffen an einen Organismus, vorzugsweise an Säuger, insbesondere Menschen, und auch Nutz- oder Haustiere.

Zu gängigen Arzneiformen gehören vor allem (in alphabetischer Reihenfolge), Emulsionen und Mikroemulsionen, Granulate, Kapseln, Pellets, Pulver, Suspensionen, Suppositorien, Tabletten, insbesondere überzogene Tabletten.

Emulsionen bzw. Mikroemulsionen können vom Öl-in-Wasser- oder Wasser-in-Öl-Typ sein und enthalten die erfindungsgemäßen Formulierungen als disperse bzw. dispergierende Phase. Zur Stabilisierung können diese Emulsionen bzw. Mikroemulsionen Emulgatoren, die bekanntermaßen zu diesem Zweck verwendet werden, enthalten. Ein Vorteil erfindungsgemäßer Formulierungen ist es allerdings, daß in der Regel nur geringe Mengen an Emulgator zugesetzt werden und gemäß einer besonderen Ausführungsform der vorliegenden Erfindung auf einen Zusatz von Emulgatoren, insbesondere von O/W-Emulgatoren mit HLB-Werten von über 10 und insbesondere von über 15 verzichtet werden kann.

Granulate bestehen aus festen Körnern erfindungsgemäßer Formulierungen, wobei jedes Korn ein Agglomerat aus Pulverpartikeln darstellt. Als Arzneiform sind Granulate vorzugsweise zur oralen Anwendung bestimmt. Dem Anwender können Einzeldosiszubereitungen, beispielsweise in einem Beutelchen (Sachet), einem Papiersack oder einem Fläschchen abgepackte Granulate, oder Mehrdosenzubereitungen, die eine entsprechende Abmessung erfordern, angeboten werden. Vielfach stellen derartige Granulate aber nicht die eigentliche Arzneiform dar, sondern sie sind Zwischenprodukte bei der Herrichtung bestimmter Arzneiformen, beispielsweise um als Tablettengranulat zu Tabletten verpreßt, als Kapselgranulat in Hartgelatinekapseln abgefüllt, oder als Trink- oder Trockensaftgranulate vor der Einnahme zunächst in Wasser gegeben zu werden.

Als Kapseln sind die erfindungsgemäßen Formulierungen in der Regel in einer harten, aus zwei Teilen zusammengesteckten oder einer weichen, einteiligen, geschlossenen Hülle von unterschiedlicher Form und Größe abgefüllt. Die Ein- oder Umhüllung bzw. Matrixeinbetturig erfindungsgemäßer Formulierungen in geeignete Polymeren, also Mikrokapseln bzw. Mikrospherulen ist ebenfalls möglich. Hart- wie Weichkapseln bestehen überwiegend aus Gelatine, wobei letztere einen geeigneten Anteil an weichmachenden Substanzen, wie Glycerol oder Sorbitol, aufweisen. Hartgelatine-Kapseln dienen zur Aufnahme erfindungsgemäßer Formulierungen, die eine feste Konsistenz aufweisen, beispielsweise als Granulat, Pulver oder Pellets. Weichgelatine-Kapseln bieten sich vor allem bei Formulierungen mit halbfester Konsistenz und erwünschtenfalls auch flüssig-viskoser Konsistenz an.

Pellets sind Granulate erfindungsgemäßer Formulierungen im Korngrößenbereich von ca. 0,5 bis 2 mm Durchmesser. Bevorzugt sind Pellets mit einer engen Korngrößenverteilung, vorzugsweise von 0,8 bis 1,2 mm sowie im wesentlichen runder Gestalt. In halbfesten Zubereitungen sind erfindungsgemäße Formulierungen in einem geeigneten Vehikel aufgenommen. Entsprechende Grundlagen sind dem Galeniker bekannt.

Suppositorien sind feste Zubereitungen zur rektalen, vaginalen oder urethralen Applikation. Um dem Verabreichungsweg gerecht zu werden, sind erfindungsgemäße Formulierungen in diesen Arzneiformen in der Regel in geeigneten Vehikeln aufgenommen, beispielsweise in bei Körpertemperatur schmelzenden Fetten wie Hartfett, Macrogole, d.h. Polyethylenglykolen mit Molekulargewichten von 1.000 bis 3.000 in verschiedenen Anteilen, Glycerolgelatine und ähnlichem.

Tabletten sind feste Zubereitungen vor allem zur oralen Anwendung. Oral besitzt im Rahmen der vorliegenden Erfindung insbesondere die Bedeutung des Begriffs "Peroral", d.h. Tabletten zur Resorption bzw. Wirkung des Wirkstoffs im Gastrointestinaltrakt. Besondere Ausführungsarten sind überzogene Tabletten, Schichttabletten, Manteltabletten, Tabletten mit modifizierter Wirkstoff-freisetzung, Matrixtabletten, Brausetabletten, Kautabletten oder Pillen. In der Regel enthalten die erfindungsgemäßen Formulierungen wenigstens einen Teil erforderlicher Tablettenhilfsstoffe, wie Bindemittel, Füllstoffe, Gleit- und Schmiermittel, bzw. Sprengmittel. Erforderlichenfalls können Tabletten erfindungsgemäße Formulierungen auch weitere geeignete Hilfsstoffe umfassen. Zu nennen sind in diesem Zusammenhang insbesondere Hilfsstoffe, welche die Tablettierung unterstützen, beispielsweise Schmier- und Gleitmittel, z.B. die oben genannten, wobei Magnesiumstearat vor allem zwecks erleichterter Komprimierung bevorzugt ist.

Überzogene Tabletten weisen darüber hinaus geeignete Überzugsmaterialien, beispielsweise Filmlacke oder Dragierhilfsmittel, vor allem die unten genannten auf. Zu den überzogenen Tabletten gehören insbesondere Dragees und Filmtabletten.

Pulver sind fein disperse Feststoffe erfindungsgemäßer Formulierungen mit Korngrößen von in der Regel weniger als 1 mm. Obige Ausführungen zu Granulaten gelten entsprechend.

Erfindungsgemäß bevorzugt sind Kapseln, die mit zerkleinertem Granulat, Pulver oder Pellets erfindungsgemäßer Formulierungen befüllt sind, Trink- und Trockensaftgranulate aus erfindungsgemäßen Formulierungen und einem Zusatz an Geschmackskorrigentien, sowie insbesondere Tabletten.

In der Regel sind die erfindungsgemäßen Arzneiformen in geeigneter Form verpackt. Durchdrückpackungen aus Kunststoff und/oder Metall für feste Arzneiformen kommen häufig zur Anwendung.

Gegenstand der vorliegenden Erfindung ist auch ein Verfahren zur Herstellung einer erfindungsgemäßen Formulierung durch Vermischen der Komponenten i), ii), iii) und gegebenenfalls iv) unter Ausbildung eines plastischen Gemisches. Somit sind für die Bildung des plastischen Gemisches wenigstens zwei Maßnahmen erforderlich, einerseits das Vermischen der gemischbildenden Komponenten, andererseits deren Plastifizieren, d.h. deren Überführung in den plastischen Zustand. Diese Maßnahmen können für einzelne oder mehrere Komponenten oder Komponententeile nacheinander, ineinandergreifend, alternierend oder in sonstiger Weise erfolgen. Demnach ist es grundsätzlich möglich, während eines Mischvorganges gleichzeitig in den plastischen Zustand zu überführen, oder zunächst zu vermischen und dann das Gemisch in den plastischen Zustand zu überführen. Im Laufe eines Verfahrens können mehrere plastische Gemische unterschiedlicher Zusammensetzung gebildet werden, die miteinander und/oder mit weiteren Komponenten oder Komponententeilen vermischt werden. Beispielsweise kann eine Vormischung aus einem Teil der Komponenten, z.B. Lipid- und Polymerkomponente, unter Ausbildung eines plastischen Gemisches granuliert werden, und das Granulat kann dann unter Zusatz weiterer Komponenten, z.B. der Wirkstoffkomponente, in ein weiteres plastisches Gemisch überführt werden, dessen Zusammensetzung derjenigen der Formulierung entsprechen kann. Es können auch sämtliche Komponenten zunächst zusammengegeben und dann entweder gleichzeitig mit dem Vermischen in den plastischen Zustand, oder zunächst vermischt und anschließend in den plastischen Zustand überführt werden.

Die Bildung eines plastischen Gemisches kann durch Aufschmelzen, oder - unter zusätzlichem Eintrag mechanischer Energie, z.B. durch Kneten, Vermischen oder Homogenisieren - auch unterhalb der Schmelztemperatur des Gemisches erfolgen. Vorzugsweise bildet man das plastische Gemisch bei Temperaturen unterhalb von 220°C. In der Regel erfolgt die Bildung des plastischen Gemischs nicht durch Anteigen oder partielles Lösen einer oder mehrerer Komponenten mit Flüssigkeiten oder Lösungsmitteln, sondern hauptsächlich oder ausschließlich durch thermische oder thermisch-mechanische Einwirkung auf die Komponente(n), d.h. durch thermisches Plastifizieren. Bevorzugt erfolgt die Bildung des plastischen Gemischs durch Extrusion, besonders bevorzugt durch Schmelzextrusion. Die Verfahrensschritte des Plastifizierens können auf an sich bekannte Art und Weise durchgeführt werden, beispielsweise wie in der EP-A-0 240 904, EP-A-0 337 256, EP-A-0358 108, WO 97/15290 und WO 97/15291 beschrieben. Auf den Inhalt dieser Publikationen und insbesondere die darin enthaltenen Ausführungen zur Schmelzextrusion wird hiermit Bezug genommen.

Die Polymerkomponente sollte sich in der Gesamtmischung aller Komponenten im Bereich von 30 bis 200°C, vorzugsweise 40 bis 170°C in einen plastischen Zustand überführen lassen. Die Glasübergangstemperatur der Mischung sollte daher unter 220°C, vorzugsweise unter 180°C liegen. Erforderlichenfalls wird sie durch übliche, pharmakologisch akzeptable weichmachende Hilfsstoffe herabgesetzt.

Beispiele für derartige Weichmacher sind:
organische, vorzugsweise schwerflüchtige Verbindungen, wie z.B. C₇-C₃₀-Alkanole, Ethylenglykol, Propylenglykol, Glycerin, Trimethylolpropan, Triethylenglykol, Butandiole, Pentanole, wie Pentaerythrit und Hexanole, Polyalkylenglykole, vorzugsweise mit einem Molekulargewicht von 200 bis 1000, wie z.B. Polyethylenglykole, Polypropylenglykole und Polyethylenpropylenglykole, Silicone, aromatische Carbonsäureester (z.B. Dialkylphthalate, Trimellithsäureester, Benzoesäureester, Terephthals-äureester) oder aliphatische Dicarbonsäureester (z.B. Dialkyladipate, Sebacinsäureester, Azelainsäureester, Zitronen- und Weinsäureester), Fettsäureester, wie Glycerinmono-, Glycerindi- oder Glycerintriacetat oder Natriumdiethylsulfosuccinat. Die Konzentration an Weichmacher beträgt soweit vorhanden im Allgemeinen 0,5 bis 30, vorzugsweise 0,5 bis 10 Gew.-%, bezogen auf das Gesamtgewicht von Polymer und Weichmacher.

Die Menge an Weichmacher beträgt vorteilhafterweise höchstens 30 Gew.-%, bezogen auf das Gesamtgewicht von Polymer und Weichmacher, damit - im Bereich fester Formen - lagerstabile Formulierungen und Arzneiformen gebildet werden, die keinen kalten Fluss zeigen. In der Regel ist der Zusatz eines Weichmachers zwecks Plastifizierung nicht erforderlich, da die erfindungsgemäß vorhandene Lipidkomponente weichmachende Eigenschaften besitzt.

Das erfindungsgemäße Verfahren kann vorteilhaft bei Temperaturen unterhalb von 200°C und bevorzugt unterhalb von 170°C, aber oberhalb von Raumtemperatur (25°C), vorzugsweise oberhalb von 40°C, durchgeführt werden. Ein bevorzugter Temperaturbereich für die Extrusion erfindungsgemäßer Formulierungen liegt bei 80 bis 150°C. Insbesondere führt man das Verfahren in einem Temperaturintervall durch, das sich 40°C, bevorzugt 30°C und besonders bevorzugt 20°C von der Erweichungstemperatur des Gemischs der Komponenten nach oben oder unten erstreckt.

In bestimmten Fällen kann es vorteilhaft sein, Komponenten oder Teile von Komponenten als Lösung oder Suspension in einem Lösungsmittel zuzugeben. Zweckmäßig sind insbesondere niedermolekulare flüchtige Lösungsmittel, z.B. Wasser, C₁-C₆-Monoalkohole und deren Ether, Ester von C₁-C₆-Monoalkanolen mit C₁-C₆-Carbonsäuren, Alkane. Ein weiteres brauchbares Lösungsmittel ist flüssiges CO₂. Wasserlösliche Wirkstoffe können als wässrige Lösung eingesetzt werden oder vorzugsweise in eine wässrige Lösung oder Dispersion der Polymerkomponente oder eines Teils davon aufgenommen werden. Entsprechendes gilt für Wirkstoffe, die in einem der genannten Lösungsmittel löslich sind, wenn die flüssige Form der zur Anwendung kommenden Komponenten auf einem organischen Lösungsmittel basiert. Die erfindungsgemäß einzusetzenden Komponenten können geringe Mengen Lösungsmittel enthalten, z.B. aufgrund von Hygroskopie, Lösungsmitteleinschlüssen oder Kristallwasser. Der Gesamtlösungsmittelgehalt des plastischen Gemisches liegt vorzugsweise unter 15%, insbesondere unter 10% und besonders bevorzugt unter 5%. Vorzugsweise erfolgt die Bildung des plastischen Gemisches ohne Zusatz eines Lösungsmittels, d.h. insbesondere durch lösungsmittelfreie Schmelzextrusion.

Die Komponenten, d.h. Wirkstoff, Lipid und Polymer sowie gegebenenfalls weitere Hilfsstoffe, können zunächst vermischt und dann in den plastischen Zustand überführt und homogenisiert werden. Hierzu können die Apparaturen; wie Rührkessel, Rührwerke, Feststoffmischer etc., im Wechsel betrieben werden. Anschließend können empfindliche Wirkstoffe eingemischt (homogenisiert) werden, vorzugsweise in "Intensivmischern" in plastischer Phase bei sehr kleinen Verweilzeiten. Der (die) Wirkstoff(e) kann (können) als solche, d.h. insbesondere in fester Form, oder als Lösung, Suspension oder Dispersion eingesetzt werden.

In bestimmten Ausführungsformen des erfindungsgemäßen Verfahrens kann es vorteilhaft sein, zunächst Wirkstoff und Lipid zu vermischen und dann zu plastifiziertem Polymer zu geben. Diese Vorgehensweise kann insbesondere dann vorteilhaft sein, wenn Wirkstoff und/oder Lipid thermisch labil sind.

Das Plastifizieren, Aufschmelzen und/oder Vermischen erfolgt in einer für diesen Zweck üblichen Vorrichtung. Besonders geeignet sind Extruder oder beheizbare Behälter mit Rührwerk, z.B. Kneter, (wie der unten noch erwähnten Art).

Als Mischapparat sind auch solche Vorrichtungen brauchbar, die in der Kunststofftechnologie zum Mischen eingesetzt werden. Geeignete Vorrichtungen sind beispielsweise beschrieben in "Mischen beim Herstellen und Verarbeiten von Kunststoffen", H. Pahl, VDI-Verlag, 1986. Besonders geeignete Mischapparaturen sind Extruder und dynamische und statische Mischer, sowie Rührkessel, einwellige Rührwerke mit Abstreifvorrichtungen, insbesondere sogenannte Pastenrührwerke, mehrwellige Rührwerke, insbesondere PDSM-Mischer, Feststoffmischer sowie vorzugsweise Misch-Knetreaktoren (z.B. ORP, CRP, AP, DTB der Firma List oder Reactotherm der Firma Krauss-Maffei oder Ko-Kneter der Fa. Buss), Doppelmuldenkneter (Trogmischer) und Stempelkneter (Innenmischer) oder Rotor/Stator-Systeme (z.B. Dispax der Firma IKA).

Die Verfahrensschritte Vermischen und Plastifizieren, also insbesondere das Aufschmelzen, können in derselben Apparatur oder in zwei oder mehreren getrennt voneinander arbeitenden Vorrichtungen ausgeführt werden. Die Zubereitung einer Vormischung kann in einer der oben beschriebenen üblichen insbesondere zur Granulierung verwendeten Mischvorrichtungen durchgeführt werden. Eine solche Vormischung kann dann direkt, z.B. in einen Extruder, eingespeist und anschließend gegebenenfalls unter Zusatz weiterer Komponenten extrudiert werden.

Das erfindungsgemäße Verfahren erlaubt es, als Extruder Einschneckenmaschinen, kämmende Schneckenmaschinen oder auch Mehrwellextruder, insbesondere Zweischnecken-Extruder, gleichsinnig oder gegensinnig drehend und gegebenenfalls mit Knetscheiben ausgerüstet, einzusetzen. Wenn bei der Extrusion ein Lösungsmittel verdampft werden muss, sind die Extruder im Allgemeinen mit einem verdampfungsteil ausgerüstet. Brauchbar sind z.B. Extruder der ZSK-Baureihe von Werner u. Pfleiderer.

Das Beschicken der Mischvorrichtung erfolgt je nach deren Konzeption kontinuierlich oder diskontinuierlich in üblicher Weise. Pulverförmige Komponenten können im freien Zulauf, z.B. über eine Differentialdosierwaage eingeführt werden. Plastische Massen können direkt aus einem Extruder eingespeist oder über eine Zahnradpumpe, die insbesondere bei hohen Viskositäten und hohen Drücken von Vorteil ist, zugespeist werden. Flüssige Medien können über ein geeignetes Pumpenaggregat zudosiert werden.

Die Lipidkomponente kann - wie vorstehend beschrieben - kontinuierlich oder diskontinuierlich in die Formulierung eingearbeitet werden. So kann wenigstens ein Teil der Lipidkomponente zunächst auf wenigstens einen Teil der Polymerkomponente (Matrix) geträgert werden, und anschließend als Vormischung unter Ausbildung eines plastischen Gemisches und eventueller Zugabe weiterer Bestandteile, vorzugsweise durch Extrusion erfindungsgemäß formuliert werden. Bevorzugt ist die kontinuierliche Zugabe wenigstens eines Teils der Lipidkomponente zu einem plastischen Gemisch. Dies ist insbesondere dann bevorzugt, wenn die erfindungsgemäß zu verwendenden Lipide in halbfester oder flüssiger Form verarbeitet werden können. Demnach sind auch aus verfahrenstechnischen Gründen die zuvor beschriebenen Lipide bevorzugt, die relativ geringe Schmelzpunkte aufweisen, von denen wiederum diejenigen bevorzugt sind, die bei Raumtemperatur, d.h. etwa 20 bis 30°C, von halbfester (wachsartige Lipide) und vorteilhafterweise von flüssiger Konsistenz (Öle) sind. Es ist bevorzugt, diese direkt in die Mischvorrichtung, insbesondere einen Extruder, zuzudosieren. Dies kann einen separat zu führenden Granulierungsschritt ersparen.

Das durch Vermischen und Überführen der Polymerkomponente, der Wirkstoffkomponente, der Lipidkomponente und gegebenenfalls weiterer Hilfsstoffe, in den plastischen Zustand erhaltene Gemisch ist teigig, zähflüssig oder dünnflüssig (thermoplastisch) und daher auch extrudierbar. Die Glasübergangstemperatur des Gemisches liegt vorteilhafterweise unter der Zersetzungstemperatur jeder in dem Gemisch enthaltenen Komponenten.

Die erfindungsgemäße Formulierung als plastisches Gemisch - gegebenenfalls nach dem Abkühlen oder Erstarren -, insbesondere als Extrudat, eignet sich für alle gängigen Verfahren zur Herrichtung gängiger Arzneiformen.

Gegenstand der vorliegenden Erfindung ist auch ein Verfahren zur Herstellung von Arzneiformen erfindungsgemäßer Formulierungen, wobei man die Formulierung nach obigem Verfahren herstellen kann und die Formulierung gegebenenfalls unter Zugabe weiterer Hilfsstoffe in die gewünschte Arzneiform bringt. Dazu kann man formgebende Verfahrensmaßnahmen anwenden, wie das Ausformen des plastischen Gemisches, insbesondere das Ausformen durch Extrusion beziehungsweise Schmelzextrusion, und das Ausformen des plastischen Gemisches, insbesondere des Extrudats - gegebenenfalls nach dem Abkühlen oder Erstarren - z.B. durch Granulieren, Mahlen, Pressen, Formgießen, Spritzgießen, Tablettieren unter Druck, Tablettieren unter Druck und Wärme. Man kann eine Formulierung auch dadurch in eine gewünschte Arzneiform bringen, daß man sie in geeignete Vehikel einbringt. So können auch an sich feste Formulierungen zu halbfesten oder flüssigen Formulierungen durch den Zusatz geeigneter Vehikel verarbeitet werden.

So lassen sich eine Vielzahl von insbesondere festen Arzneiformen herrichten. Beispielsweise kann man durch Mahlen oder Zerhacken des erstarrten oder zumindest teilweise erstarrten plastischen Gemischs Pulver oder Granulate herstellen, die entweder direkt zur Therapierung eingesetzt oder gegebenenfalls unter Zugabe üblicher Hilfsstoffe zu obigen Arzneiformen, vor allem zu Tabletten, weiterverarbeitet werden können.

Vorzugsweise werden Arzneiformen vor dem Erstarren des plastischen Gemischs geformt, die, gegebenenfalls nach Beschichten, Coaten, Dragieren oder mit einem Film überzogen, in therapeutisch einsetzbarer Form anfallen.

Die Formung zur Arzneiform vor dem Erstarren kann in Abhängigkeit von der Viskosität des plastischen Gemischs auf vielfältige Weise erfolgen, z.B. durch Formgießen, Spritzgießen, Pressen, Quetschen oder Kalandrieren. Dazu wird das vorstehend beschriebene plastische Gemisch im erfindungsgemäßen Verfahren einem oder mehreren Formungsschritten zugeführt. Das Zuführen kann durch Pressen, Pumpen, z.B. mit Zahnradpumpen, oder vorzugsweise mit einem Extruder erfolgen.

Besonders bevorzugt wird das plastische Gemisch in einem oder mehreren, bevorzugt einem, Extruder gebildet und mit diesem oder einem nachgeschalteten Extruder den Formungsschritten zugeführt. In vielen Fällen hat es sich als vorteilhaft herausgestellt, schräg abwärts zu extrudieren und/oder gegebenenfalls eine Führungsrinne zum Transport des Extrudats vorzusehen, um einen sicheren Transport zu gewährleisten und ein Abreißen des extrudierten Stranges zu vermeiden.

In Abhängigkeit von der Anzahl und Verträglichkeit der einzusetzenden Wirkstoffe können vorteilhaft auch mehrschichtige Extrudate, z.B. Coextrudate, wie in der WO 96/19963 beschrieben, mitdem erfindungsgemäßen Verfahren verarbeitet werden.

Mehrschichtige feste Arzneiformen können insbesondere durch Koextrusion hergestellt werden, wobei mehrere Gemische aus einzelnen oder mehreren der oben beschriebenen Komponenten bei der Extrusion so in einem Werkzeug zusammengeführt werden, dass sich der gewünschte Schichtaufbau ergibt. Vorzugsweise verwendet man verschiedene Polymere für verschiedene Schichten.

Mehrschichtige Arzneiformen umfassen vorzugsweise zwei oder drei Schichten. Sie können in offener oder geschlossener Form vorliegen, insbesondere als offene oder geschlossene Mehrschichttabletten.

Erfolgt das Ausformen durch Koextrusion, so werden die Gemische aus den einzelnen Extrudern oder anderen Aggregaten in ein gemeinsames Koextrusionswerkzeug geführt und ausgetragen. Die Form der Koextrusionswerkzeuge richtet sich nach der gewünschten Arzneiform. Beispielsweise sind Werkzeuge mit ebenem Austrittsspalt, sogenannte Breitschlitzwerkzeuge, und Werkzeuge mit kreisringspaltförmigem Austrittsquerschnitt geeignet. Die Düsenauslegung erfolgt dabei in Abhängigkeit von der zur Anwendung kommenden Formulierungsgrundlage, und insbesondere der Polymerkomponente und der gewünschten Arzneiform.

Der erste Formungsschritt erfolgt vorteilhaft beim Austrag des Extrudats aus dem Extruder durch geeignet geformte Düsen, Blenden oder sonstige Austrittsöffnungen, z.B. durch eine Lochblende, eine Runddüse oder eine Breitschlitzdüse. In der Regel wird so kontinuierlich ein Strangextrudat mit vorzugsweise konstantem Querschnitt, z.B. in Form eines Bandes oder eines Stranges, vorzugsweise mit rundem, ovalem, abgerundetem oder flachem und breitem Querschnitt, erhalten.

Geeignete nachgeschaltete Formungsschritte für Extrudate sind z.B. der Kaltabschlag, d.h. das Schneiden beziehungsweise Zerhakken des Stranges nach zumindest teilweisem Erstarren, der Heißabschlag, d.h. das Zerschneiden beziehungsweise Zerhacken des Stranges in noch plastischer Form oder das Abquetschen des noch plastischen Strangs in einer Quetschvorrichtung. Mit Heiß- oder Kaltabschlag lassen sich z.B. Granulate (Heiß- oder Kaltgranulierung) oder Pellets erhalten. Die Heißgranulierung führt in der Regel zu Arzneiformen (Pellets) mit einem Durchmesser von 0,5 bis 3 mm, während die Kaltgranulierung normalerweise zu zylinderförmigen Produkten mit einem Verhältnis von Länge zu Durchmesser von 1 bis 10 und einem Durchmesser von 0,5 bis 10 mm führt. So können einschichtige, bei Anwendung der Koextrusion aber auch offene oder geschlossene, mehrschichtige Arzneiformen hergestellt werden, beispielsweise Oblongtabletten, Pastillen und Pellets. Die Arzneiformen können in einem nachgeschalteten Verfahrensschritt nach üblichen Methoden mit einem Coating versehen werden. Geeignete Materialien für Filmüberzüge sind die als polymere Bindemittel genannten Polymere, insbesondere Polyacrylate, wie die Eudragit®-Typen, Celluloseester, wie die Hydroxypropylcellulosephthalate, sowie Celluloseether, wie Ethylcellulose, Hydroxypropylmethylcellulose oder Hydroxypropylcellulose und Gelatine. Auch weitere Formungsschritte können sich anschließen, wie z.B. die Arrondierung oder Verrundung der durch den Heiß- oder Kaltabschlag erhaltenen Pellets mittels Arrondiervorrichtungen, wie in der DE-A-196 29 753 beschrieben.

Besonders bevorzugt werden alle Formungsschritte am noch plastischen Gemisch bzw. noch plastischen Extrudat durchgeführt. Neben dem Heißabschlag, gegebenenfalls mit nachfolgendem Arrondieren, eignet sich insbesondere ein Verfahren, bei dem man das plastische Gemisch in einem Formkalander zur Dosierungsform formt. Dazu wird ein noch plastisches Gemisch oder ein noch plastisches Extrudat einem geeigneten Formkalander zugeführt. Geeignete Formkalander weisen zur Formung in der Regel Formwalzen und/oder Bänder auf, wobei mindestens eine der Formwalzen und/oder mindestens eines der Bänder Vertiefungen zur Aufnahme und Formung des plastischen Gemischs aufweist. Vorzugsweise verwendet man einen Formkalander mit gegenläufig rotierenden Formwalzen, wobei mindestens eine der Formwalzen auf ihrer Oberfläche Vertiefungen zur Aufnahme und Formung des plastischen Gemischs aufweist. Geeignete Formkalander und Formwalzen enthaltende Vorrichtungen sind allgemein beispielsweise in der EP-A-0 240 904, EP-A-0 240 906 und WO 96/19962-, geeignete Bänder und Bänder enthaltende Vorrichtungen allgemein beispielsweise in der EP-A-0 358 105 offenbart, auf die diesbezüglich hiermit Bezug genommen wird.

Die Formgebung des noch plastischen Gemischs oder noch plastischen Extrudats erfolgt vorzugsweise bei Schmelztemperaturen unterhalb von 220°C, besonders bevorzugt unterhalb von 180°C und ganz besonders bevorzugt unterhalb von 150°C, wie z.B. in den zur Bildung des plastischen Gemischs notwendigen Temperaturbereichen oder bei niedrigeren Temperaturen. Wenn die Formung bei niedrigeren Temperaturen erfolgt, erfolgt sie vorteilhaft 5 bis 70°C, bevorzugt 10 bis 50°C und besonders bevorzugt 15 bis 40°C unterhalb der höchsten bei der Bildung des plastischen Gemischs erreichten Temperatur, vorzugsweise jedoch oberhalb der Erstarrungstemperatur des plastischen Gemischs.

Die erfindungsgemäße Herstellung der Formulierungen und Zubereitung der Arzneiformen kann ganz oder teilweise unter sterilen Arbeitsbedingungen durchgeführt werden, z.B. in Reinräumen und unter Verwendung sterilisierter Geräte, wie z.B. Waagen, Mischern, Extrudern und Formungsmaschinen, wie Kalandern, Quetschvorrichtungen und Zerhackern. Die Einsatzstoffe können entweder in sterilisierter Form, gegebenenfalls unter Zugabe geeigneter antibakterieller und/oder antiviraler Hilfsstoffe, in das Verfahren eingebracht werden und/oder die Verfahrensbedingungen, insbesondere die Temperatur, so gewählt werden, dass sterile Formulierungen bzw. Arzneiformen erhalten werden. Die erhaltenen sterilen Dosierungsformen können anschließend unter ebenfalls sterilen Bedingungen direkt verpackt werden, z.B. durch Verblistern oder Einschweißen. Die Formgebung und das Verpacken kann auch gleichzeitig durchgeführt werden, insbesondere wenn die Formgebung des plastischen Gemischs durch Kalandrieren mittels Formwalzen durchgeführt wird. Dazu bringt man zusätzlich zu dem plastischen Gemisch als Folien vorliegende Materialien jeweils zwischen Schmelze und Formwalze, wodurch gleichzeitig mit der Formung des plastischen Gemischs zu Dosierungsformen eine Umhüllung und/oder eine Verpackung der Dosierungsform erreicht werden kann, wie in der WO-96/19963 beschrieben, auf die diesbezüglich hiermit Bezug genommen wird.

Ein weiterer Gegenstand der vorliegenden Erfindung ist die Verwendung einer erfindungsgemäßen Formulierungsgrundlage bei der Verabreichung und insbesondere der oralen Verabreichung wenigstens eines wasserlöslichen Wirkstoffs, insbesondere eines Heparins, Glycosaminoglykans oder Heparinoids. Zweck dieser Verwendung ist es insbesondere, die pharmakologische Wirkung der Wirkstoffkomponente zu verbessern. So beinhaltet diese Verwendung insbesondere ein Verfahren zur Verbesserung der pharmakologischen Wirkung der Wirkstoffkomponente bei der Verabreichung und insbesondere der oralen Verabreichung wenigstens eines Heparins, Glycosaminoglykans oder Heparinoids, wobei man eine erfindungsgemäße Formulierungsgrundlage vewendet. Dabei bringt man wenigstens ein Heparin, Glycosaminoglykan oder Heparinoid in diese Formulierungsgrundlage ein, vorzugsweise mit einem der vorstehend geschilderten Verfahren. Insbesondere dient die Polymermatrix der Formulierungsgrundlage zur Aufnahme wenigstens eines Lipids bei der Herstellung einer erfindungsgemäßen festen Formulierung zur Verbesserung der pharmakologischen Wirkung der Wirkstoffkomponente. Die Verbesserung der pharmakologischen Wirkung greift insbesondere bei der oralen Verabreichung der Heparin, Glycosaminoglykan und/oder Heparinoid enthaltenden Formulierungsgrundlage an einen Säuger, insbesondere einen Menschen, ein Nutz- oder Haustier.

Die Polymermatrix wird von der zuvor beschriebenen Polymerkomponente oder zumindest eines Teils davon gebildet. In diese Polymermatrix wird wenigstens ein Lipid, das Bestandteil der zuvor beschriebenen Lipidkomponente ist, aufgenommen. Insbesondere bevorzugt ist es, dass die Aufnahme zu einer im Wesentlichen molekulardispersen Verteilung von Lipid in der Polymermatrix führt. Eine homogene Verteilung von Lipid in der Matrix ist von Vorteil, insbesondere im Hinblick auf wirkstoffördernde Eigenschaften des Lipids. Diese Vorteile können auch erzielt werden, ohne den Wirkstoff molekulardispers zu verteilen. Lipide, die zur Verbesserung der pharmakologischen Wirkung des Wirkstoffs dienen können, sind dem Fachmann u.a. als Resorptionsförderer bekannt. Unter diesen kann er beispielsweise wenigstens einen Teil der Lipidkomponente auswählen. Ergänzend wird auf die Ausführungen oben in Zusammenhang mit der Beschreibung der Lipidkomponente Bezug genommen.

Die erfindungsgemäße Verwendung ist insbesondere immer dann von Vorteil, wenn Wirkstoffe auf eine Art und Weise verabreicht werden sollen, bei der durch gleichzeitige Gabe von Lipiden ein wirkstoffördernder Effekt auftreten kann. Dies betrifft vor allem Verabreichungswege, die den Gastrointestinaltrakt mit einbeziehen, also insbesondere die enterale, vor allem rektale und vorzugsweise orale Verabreichung. Ganz besonders von Vorteil ist die erfindungsgemäße Anwendung dann, wenn ein zu verabreichender Wirkstoff ohne geeignete Maßnahmen, wie dem Zusatz wenigstens eines Lipids, auf diesem Wege nur unzureichend angewendet werden kann. Dies ist der Fall bei wasserlöslichen Wirkstoffen, wie Heparinen, Glycosaminoglykanen und Heparinoiden.

Erfindungsgemäße Arzneiformen werden dem zu behandelnden Individuum, vorzugsweise einem Säuger, insbesondere einem Menschen, und auch Nutz- oder Haustier, und damit eine wirksame Menge an Wirkstoff verabreicht. Ob eine solche Behandlung angezeigt ist und in welcher Form sie zu erfolgen hat, hängt vom Einzelfall ab und unterliegt einer medizinischen Beurteilung (Diagnose), die vorhandene Anzeichen, Symptome und/oder Fehlfunktionen, Risiken bestimmte Anzeichen, Symptome und/oder Fehlfunktionen zu entwikkeln, und weitere Faktoren mit einbezieht. In der Regel werden die erfindungsgemäßen Arzneiformen einmal oder mehrmals am Tag zusammen oder im Wechsel mit anderen. Präparaten derart verabreicht, daß einem zu behandelnden Individuum eine Tagedosis in einer Menge zugeführt wird, die eine Therapie ermöglicht.

Erfindungsgemäße Formulierungen finden vornehmlich Anwendung als Antikoagulantia. Hierzu gehören die Prophylaxe und Therapie thromboembolischer Erkrankungen, beispielsweise arterieller und venöser Thrombosen und Embolien, Früh- und Langzeitbehandlungen des Herzinfarktes, insbesondere bei erhöhtem Risiko für thromboembolische Komplikationen, prä- und postoperative Thromboseprophylaxe zur Vermeidung von Thrombosen im extrakorporalen Kreislauf, beispielsweise während der Hämodialyse und Hämofiltration, Verbrauchskoagulopathie, insbesondere in der hyperkoagulatorischen Phase, und ähnliche Zustände, bei denen eine die Blutgerinnung hemmende Wirkung angezeigt ist. Weitere Indikationsfelder sind beispielsweise tiefe Venenthrombosen, Lungenembolie, kardiovaskuläre Erkrankungen, instabile Angina, Myokardinfarkt, Gehirnschlag, Arrhythmie, Entzündungen, rheumatoide Arthritis, Crohn-Krankheit, endzündliche Darmerkrankungen, diabetische Retinopathie, diabetische Nephropathie, Transplantatabstoßung, Angiogeneseshemmung, Metastasenhemmung und Krebs.

Die vorliegende Erfindung soll nun anhand der folgenden Beispiele veranschaulicht werden.

Es zeigt die
Figur 1 die Konzentration an Heparin, die an das Endothel der Vena Cava bzw. der Aorta abdominalis männlicher Wistar-Ratten nach intragastraler Applikation von Placebo (0), niedermolekularem Heparin (Reviparin), und der erfindungsgemäßen Formulierungen E1 und E2;
Figur 2 die Inzidenz von Thrombosen an männlichen Wistar-Ratten nach Applikation von Placebo (0), niedermolekularem Heparin (Reviparin), und den erfindungsgemäßen Formulierungen E1 und E2.

### Beispiel 1:

In einem Labor-Doppelschnecken-Extruder (Fa. Haake, 16 mm Schnekkendurchmesser) wurde ein Gemisch aus 20 Gew.-% LMWH (niedermolekulares Heparin, Molekulargewichtsverteilung 2000 bis 10000; Reviparin), 64 Gew.-% Kollidon®VA-64, 16 Gew.-% Ölsäure und 1 Gew.-% Aerosil 200 bei einer Temperatur von 110°C zu einer weißen, homogenen Schmelze extrudiert, die nach dem Abkühlen in einer Labormühle gemahlen wurde. Das erhaltene Pulvergranulat E1 löste sich unter Bildung einer Emulsion in Wasser. Die Größe der Emulsionströpfchen dieser Zubereitung wurde mit Hilfe eines Mastersizer-Geräts (Fa. Malvern, UK) gemessen. 90 % der Partikel wiesen Größen von unter 25 µm auf, 50 % der Partikel waren kleiner als 1,8 µm (bimodale Verteilung).

### Beispiel 2:

Analog Beispiel 1 wurde ein Gemisch aus 20 Gew.-% LMWH (niedermolekulares Heparin, Molekulargewichtsverteilung 2000 bis 10000; Reviparin), 70 Gew.-% Hydroxypropylcellulose (Klucel® EF), 10 Gew.-% Ölsäure und 1 Gew.-% Aerosil 200 bei einer Temperatur von 130°C extrudiert. Es wurde eine weiße, nach dem Abkühlen feste Schmelze erhalten, die in einer Labormühle zu einem Pulvergranulat E2 gemahlen wurde, das in Wasser unter Bildung einer Emulsion löslich war. Die Größe der Emulsionströpfchen dieser Zubereitung wurde mit Hilfe eines Mastersizer-Geräts (Fa. Malvern, UK) gemessen. 90 % der Partikel wiesen Größen von unter 32 µm auf, 50 % der Partikel waren kleiner als 12 µm.

### Beispiel 3:

Analog Beispiel 1 jedoch mit 20 Gew.-% Dextransulfat (Fa. ICN) anstelle von LMWH wurde bei 110°C extrudiert; der so erhaltene elfenbeinfarbene Extrudatstrang wurde nach dem Abkühlen in einer Mühle gemahlen.

### Beispiel 4:

Analog Beispiel 2 jedoch mit 20 Gew.-% Dextransulfat (Fa. ICN) anstelle von LMWH wurde bei 150°C extrudiert; der so erhaltene leicht gelbliche Extrudatstrang wurde nach dem Abkühlen in einer Mühle gemahlen.

### Beispiel 5:

Ein Gemisch aus 20 Gew.-% Palmitinsäure und 20 Gew.-% Ölsäure wurde in einem beheizten Kessel bei einer Temperatur von. 70°C vollständig verflüssigt, und es wurden 3 Gew.-% Hydroxypropylcellulose und 57 Gew.-% niedermolekulares Heparin homogen unter Rühren/Kneten eingearbeitet. Das Gemisch wurde noch warm in Hartgelatine-Kapseln abgefüllt.

### Beispiel 6:

Das in den Beispielen 1 und 2 eingearbeitete Reviparin besitzt ursprünglich eine spezifische Anti-Xa-Aktivität von 136 IU/mg.
Die anschließende Überprüfung des Gehalts an Reviparin im Extrudat erfolgte mittels HPLC, die biologische Aktivität des extrudierten Reviparins, gemessen als Hemmwirkung auf den Blutgerinnungsfaktor Xa, erfolgte nach TEIEN, A.N. et al: Assay of heparin in plasma using a chromogenic substrate, Thromb. Res. 8, 413-416 (1976). So ergaben sich für die Extrudate E1 und E2 bei einem Wirkstoffgehalt von 23,4 % bzw. 17,7 % Aktivitäten von 31,3 IU/mg bzw. 24,1 IU/mg. Dies entspricht 98 bzw. 100 % der ursprünglichen Aktivität.

### Beispiel 7:

Die Schmelzextrudate E1 oder E2, Reviparin oder Placebo (Kochsalzlösung) wurden männlichen Wistar-Ratten mittels Schlundsonde intragastral appliziert. Pro Substanzgruppe wurden 20 Tiere verwendet. Die Dosis der Wirksubstanz betrug jeweils 0,025 mg/kg Körpergewicht. 4 Stunden nach Verabreichung der Testsubstanzen wurden den Tieren unter Narkose die Vena cava und die Aorta abdominalis entnommen. Das Endothel der Blutgefäße wurde nach Hiebert and Jaques (Artery, 2, 26, 1976) aufgearbeitet. Der Gehalt an endothelgebundenem Reviparin wurde mittels Agarose-Gel-Elektrophorese bestimmt, wie bei Jaques et al. (J Lab Clin Med, 115, 422, 1990) beschrieben. Es wurden nach Gabe von E1 und E2 signifikant höhere Konzentrationen an Reviparin am Endothel gefunden.

### Beispiel 8:

Männliche Wistar-Ratten wurden kurzzeitig narkotisiert. Nach Eröffnung der Haut oberhalb der Vena jugularis wurden 5 Tropfen einer Formalin/Methanol-Lösung appliziert (10/65 Vol.-%). Dadurch wird, wie bei Blake et al. (J Clin Path, 12, 118, 1959) beschrieben, eine Thrombose chemisch induziert. 4 Stunden nach Thromboseinduktion wurde die Jugularvene auf Vorhandensein eines harten Gerinnsels untersucht. Pro Gruppe wurden 20 Tiere verwendet. Die Schmelzextrudate E1 oder E2, Reviparin oder Plazebo (Kochsalzlösung) wurden den Versuchstieren mittels Schlundsonde 24 Stunden vor Thrombusinduktion intragastral appliziert. Die Dosis betrug 7,5 mg/kg Körpergewicht.

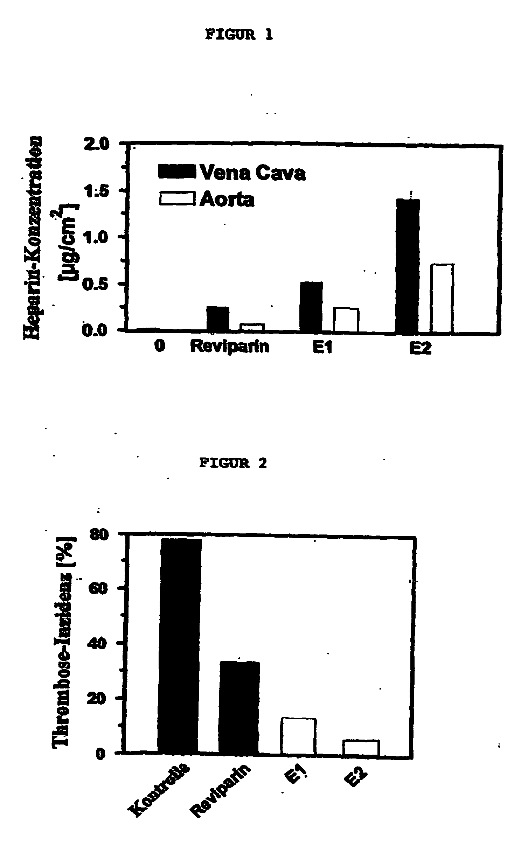

Zur Auswertung wurde die Inzidenz von Thrombosen pro Gruppe herangezogen (Figur 2). Es wurde gefunden, daß Hemmung der Thrombusbildung nach Gabe von den Schmelzextrudaten E1 und E2 deutlich höher war als nach Gabe von Reviparin allein.

## Patentansprüche

1. Feste oder halbfeste Formulierung, umfassend
i) 5-40 Gew.-% einer aus wenigstens einem Heparin, Glycosaminoglykan oder Heparinoid und gegebenenfalls wenigstens einem weiteren Wirkstoff gebildeten Wirkstoffkomponente;
ii) eine aus wenigstens einem Lipid gebildete Lipidkomponente, wobei die Lipidkomponente einen HLB-Wert von höchstens 12 aufweist;
iii) 10 - 80 Gew.-% einer aus einem oder mehren unter Polyvinylpyrrolidonen, Vinylpyrrolidon-Vinylacetat-Copolymeren, Hydroxyalkylcellulosen, Hydroxyalkyl-Alkylcellulosen, Cellulosephthalaten und (Meth)acrylharzen ausgewählten Polymer(en) gebildeten festen Polymerkomponente; und
iv) gegebenenfalls wenigstens einen weiteren pharmazeutisch akzeptablen Hilfsstoff.
wobei die Formulierung weniger als 1 Gew.-% Wasser umfasst.

2. Formulierung nach Anspruch 1, **dadurch gekennzeichnet, dass** das Heparin ein niedermolekulares Heparin ist.

3. Formulierung nach Anspruch 1 oder 2, **dadurch gekennzeichnet, dass** der Anteil an Wirkstoffkristallen weniger als 5 % beträgt.

4. Formulierung nach Anspruch 3, **dadurch gekennzeichnet, dass** die Formulierung im wesentlichen frei von Wirkstoffkristallen ist.

5. Formulierung nach einem der Ansprüche 1 bis 4, **dadurch gekennzeichnet, dass** wenigstens ein Lipid der Lipidkomponente ausgewählt ist unter Fettsäuren, Triglyceriden, Diglyceriden und Monoglyceriden.

6. Formulierung nach Anspruch 5, **dadurch gekennzeichnet, dass** die Fettsäure ungesättigt ist.

7. Formulierung nach einem der Ansprüche 1 bis 6, **dadurch gekennzeichnet, dass** die Lipidkomponente einen HLB-Wert von höchstens 8 aufweist.

8. Formulierung nach einem der Ansprüche 1 bis 7, **dadurch gekennzeichnet, dass** die Lipidkomponente einen Schmelzpunkt von weniger als 30 °C aufweist.

9. Formulierung nach einem der Ansprüche 1 bis 8, **dadurch gekennzeichnet, dass** wenigstens ein Teil der Lipidkomponente in molekulardisperser Form vorliegt.

10. Formulierung nach einem der Ansprüche 1 bis 9, **dadurch gekennzeichnet, dass** sie 3 bis 50 Gew.-% Lipidkomponente enthält.

11. Formulierung nach einem der Ansprüche 1 bis 10, **dadurch gekennzeichnet, dass** der Anteil der Lipidkomponente bezogen auf die Polymerkomponente höchstens 40 Gew.-% beträgt.

12. Formulierung nach einem der Ansprüche 1 bis 11, **dadurch gekennzeichnet, dass** wenigstens ein Teil der Lipidkomponente molekulardispers in der Polymerkomponente verteilt ist.

13. Formulierung nach einem der Ansprüche 1 bis 12, **dadurch gekennzeichnet, dass** die Formulierung weniger als 1 Gew.-% oberflächenaktiven Hilfsstoff mit einem HLB-Wert von mehr als 15 enthält.

14. Formulierung nach einem der Ansprüche 1 bis 13, **dadurch gekennzeichnet, dass** sie fest ist.

15. Feste Formulierung mit einem Wassergehalt von weniger als 1 Gew.-%, enthaltend
i) 5 - 40 Gew.-% niedermolekulares Heparin mit einem gewichtsmittleren Molekulargewicht von etwa 500 bis etwa 10000;
ii) wenigstens eine ungesättigte Fettsäure, die ausgewählt ist unter Ölsäure, Linolsäure und/oder Linolensäure, oder entsprechenden Mono- oder Diglyceriden;
iii) 10 - 80 Gew.-% einer aus einem oder mehreren unter Polyvinylpyrrolidonen, Vinylpyrrolidon-Coppolymerisaten mit Vinylacetat, Hydroxypropylcellulosen oder Hydroxypropylmethylcellulosen ausgewählten Polymer(en) gebildeten Polymerkomponente; und
iv) gegebenenfalls wenigstens einen weiteren Hilfsstoff.

16. Formulierung nach einem der Ansprüche 1 bis 15, **dadurch gekennzeichnet, dass** der weitere Hilfsstoff ausgewählt ist unter Füllstoffen, Schmiermitteln, Gleitmitteln, Trennmitteln, Fließmitteln, Farbstoffen, Stabilisatoren, Weichmachern, Konservierungsmitteln, Sprengmitteln, Adsorptionsmitteln, Formentrennmitteln und Tensiden.

17. Formulierung nach einem der Ansprüche 1 bis 16, **dadurch gekennzeichnet, dass** sie durch Schmelzextrusion eines die Komponenten i), ii), iii) und gegebenenfalls iv) enthaltenden Gemisches erhältlich ist.

18. Formulierung nach einem der Ansprüche 1 bis 17, zur Verwendung als Arzneiform zur oralen Verabreichung wenigstens eines Heparins, Glycosaminoglykans oder Heparinoids.

## Claims

1. A solid or semisolid formulation comprising
i) 5-40% by weight of an active ingredient component formed from at least one heparin, glycosaminoglycan or heparinoid and optionally at least one further active ingredient;
ii) a lipid component formed from at least one lipid, where the lipid component has an HLB not exceeding 12;
iii) 10-80% by weight of a solid polymer component formed from one or more polymer(s) selected from polyvinylpyrrolidones, vinylpyrrolidonevinyl acetate copolymers, hydroxyalkylcelluloses, hydroxyalkylalkylcelluloses, cellulose phthalates and (meth)acrylic resins; and
iv) optionally at least one further pharmaceutically acceptable excipient,
where the formulation comprises less than 1% by weight of water.

2. The formulation according to claim 1, wherein the heparin is a low molecular weight heparin.

3. The formulation according to claim 1 or 2, wherein the content of active ingredient crystals is less than 5%.

4. The formulation according to claim 3, wherein the formulation is substantially free of active ingredient crystals.

5. The formulation according to any of claims 1 to 4, wherein at least one lipid of the lipid component is selected from fatty acids, triglycerides, diglycerides and monoglycerides.

6. The formulation according to claim 5, wherein the fatty acid is unsaturated.

7. The formulation according to any of claims 1 to 6, wherein the lipid component has an HLB not exceeding 8.

8. The formulation as claimed in any of claims 1 to 7, wherein the lipid component has a melting point of less than 30°C.

9. The formulation according to any of claims 1 to 8, wherein at least part of the lipid component is in the form of a molecular dispersion.

10. The formulation according to any of claims 1 to 9, which comprises from 3 to 50% by weight of lipid component.

11. The formulation according to any of claims 1 to 10, wherein the content of the lipid component based on the polymer component does not exceed 40% by weight.

12. The formulation according to any of claims 1 to 11, wherein at least part of the lipid component is in the form of a molecular dispersion in the polymer component.

13. The formulation according to any of claims 1 to 12, wherein the formulation comprises less than 1% by weight of surface-active excipient with an HLB of more than 15.

14. The formulation according to any of claims 1 to 13, which is solid.

15. A solid formulation with a water content of less than 1% by weight, comprising
i) 5-40% by weight of low molecular weight heparin with a weight average molecular weight of about 500 to about 10 000;
ii) at least one unsaturated fatty acid which is selected from oleic acid, linoleic acid and/or linolenic acid, or corresponding mono- or diglycerides;
iii) 10-80% by weight of a polymer component formed from one or more polymer(s) selected from polyvinylpyrrolidones, vinylpyrrolidone copolymers with vinyl acetate, hydroxypropylcelluloses or hydroxypropylmethylcelluloses; and
iv) optionally at least one further excipient.

16. The formulation according to any of claims 1 to 15, wherein the further excipient is selected from fillers, lubricants, glidants, separating agents, flow regulators, dyes, stabilizers, plasticizers, preservatives, disintegrants, adsorbents, mould release agents and surfactants.

17. The formulation according to any of claims 1 to 16, which is obtainable by melt extrusion of a mixture comprising components i), ii), iii) and optionally iv).

18. The formulation according to any of claims 1 to 17, for use as drug form for oral administration of at least one heparin, glycosaminoglycan or heparinoid.

## Revendications

1. Formulation solide ou semi-solide, comprenant
i) 5 à 40 % en poids d'un composant de principe actif constitué d'au moins une héparine, un glycosaminoglycane ou un héparinoïde et le cas échéant d'au moins un autre principe actif ;
ii) un composant lipidique constitué d'au moins un lipide, le composant lipidique présentant une valeur HLB d'au maximum 12;
iii) 10 à 80 % en poids d'un composant polymère solide formé à partir d'un ou de plusieurs polymères choisis parmi les polyvinylpyrrolidones, les copolymères de vinylpyrrolidone - acétate de vinyle, les hydroxyalkylcelluloses, les hydroxyalkylalkylcelluloses, les phtalates de cellulose et les résines (méth)acryliques ; et
iv) le cas échéant au moins un autre auxiliaire pharmaceutiquement acceptable ; et
la formulation comprenant moins de 1 % en poids d'eau.

2. Formulation selon la revendication 1, **caractérisée en ce que** l'héparine est une héparine de faible poids moléculaire.

3. Formulation selon la revendication 1 ou 2, **caractérisée en ce que** la proportion de cristaux de principe actif est inférieure à 5 %.

4. Formulation selon la revendication 3, **caractérisée en ce que** la formulation est sensiblement exempte de cristaux de principe actif.

5. Formulation selon l'une des revendications 1 à 4, **caractérisée en ce qu'**au moins un lipide du composant lipidique est choisi parmi les acides gras, les triglycérides, les diglycérides et les monoglycérides.

6. Formulation selon la revendication 5, **caractérisé en ce que** l'acide gras est insaturé.

7. Formulation selon l'une des revendications 1 à 6, **caractérisée en ce que** le composant lipidique présente une valeur HLB d'au maximum 8.

8. Formulation selon l'une des revendications 1 à 7, **caractérisée en ce que** le composant lipidique présente un point de fusion de moins de 30°C.

9. Formulation selon l'une des revendications 1 à 8, **caractérisée en ce qu'**au moins une partie du composant lipidique se présente sous forme dispersée sur le plan moléculaire.

10. Formulation selon l'une des revendications 1 à 9, **caractérisée en ce qu'**elle comprend 3 à 50 % en poids du composant lipidique.

11. Formulation selon l'une des revendications 1 à 10, **caractérisée en ce que** la proportion du composant lipidique par rapport au composant polymère atteint au maximum 40 % en poids.

12. Formulation selon l'une des revendications 1 à 11, **caractérisée en ce qu'**au moins une partie du composant lipidique est répartie de manière dispersée sur le plan moléculaire dans le composant polymère.

13. Formulation selon l'une des revendications 1 à 12, **caractérisée en ce que** la formulation contient moins de 1 % en poids d'auxiliaire tensitiactif ayant une valeur HLB de plus de 15.

14. Formulation selon l'une des revendications 1 à 13, **caractérisée en ce qu'**elle est solide.

15. Formulation solide ayant une teneur en eau de moins de 1 % en poids, contenant
i) 5 à 40 % en poids d'héparine de faible poids moléculaire, ayant un poids moléculaire moyen en poids d'environ 500 à environ 10000 ;
ii) au moins un acide gras insaturé qui est choisi parmi l'acide oléique, l'acide linolique et/ou l'acide linolénique ou les monoglycérides ou diglycérides correspondants ;
iii) 10 à 80 % en poids d'un composant polymère formé à partir d'un ou de plusieurs polymères choisis parmi les polyvinylpyrrolidones, les copolymères de vinylpyrrolidone avec de l'acétate de vinyle, les hydroxypropylcelluloses ou les hydroxypropylméthylcelluloses ; et
iv) le cas échéant au moins un autre auxiliaire.

16. Formulation selon l'une des revendications 1 à 15, **caractérisée en ce que** l'autre auxiliaire est choisi parmi les charges, les lubrifiants, les agents de glissement, les agents séparateurs, les agents d'écoulement, les colorants, les stabilisateurs, les plastifiants, les conservateurs, les agents d'éclatement, les agents d'adsorption, les agents de démoulage et les tensioactifs.

17. Formulation selon l'une des revendications 1 à 16, **caractérisée en ce qu'**elle peut être obtenue par extrusion fusion d'un mélange contenant les composants i), ii), iii) et le cas échéant iv).

18. Formulation selon l'une des revendications 1 à 17 destinée à l'utilisation sous forme pharmaceutique pour l'administration orale d'au moins une héparine, un glycosaminoglycane ou un héparinoïde.
